(19) Europäisches Patentamt / European Patent Office / Office européen des brevets

(11) EP 1 699 815 B1

(12) **FASCICULE DE BREVET EUROPEEN**

(45) Date de publication et mention de la délivrance du brevet:
**06.10.2010 Bulletin 2010/40**

(21) Numéro de dépôt: **04816418.0**

(22) Date de dépôt: **17.12.2004**

(51) Int Cl.:
***C07K 7/02*** *(2006.01)* ***C07K 7/06*** *(2006.01)*

(86) Numéro de dépôt international:
**PCT/FR2004/003283**

(87) Numéro de publication internationale:
**WO 2005/061530 (07.07.2005 Gazette 2005/27)**

(54) **NOUVEAUX MODULATEURS DU PROTEASOME**

NEUE PROTEASOMMODULATOREN

NOVEL PROTEASOME MODULATORS

(84) Etats contractants désignés:
**AT BE BG CH CY CZ DE DK EE ES FI FR GB GR HU IE IS IT LI LT LU MC NL PL PT RO SE SI SK TR**

(30) Priorité: **18.12.2003 FR 0314958**

(43) Date de publication de la demande:
**13.09.2006 Bulletin 2006/37**

(73) Titulaire: **CENTRE NATIONAL DE LA RECHERCHE SCIENTIFIQUE (CNRS)**
**75016 Paris (FR)**

(72) Inventeurs:
- **REBOUD-RAVAUX, Michèle**
  **F-75013 PARIS (FR)**
- **BERNARD, Elise**
  **F-70400 CHAVANNE (FR)**
- **PAPAPOSTOLOU, David**
  **F-94120 FONTENAY SOUS BOIS (FR)**
- **VANDERESSE, Régis**
  **F-54840 SEXEY LES BOIS (FR)**

(74) Mandataire: **Corizzi, Valérie et al**
**Cabinet ORES,**
**36, rue de Saint Pétersbourg**
**75008 Paris (FR)**

(56) Documents cités:
**WO-A-98/33812**

- **PAPAPOSTOLOU DAVID ET AL: "Regulation of the 26S proteasome activities by peptides mimicking cleavage products" BIOCHEMICAL AND BIOPHYSICAL RESEARCH COMMUNICATIONS, vol. 295, no. 5, 2 août 2002 (2002-08-02), pages 1090-1095, XP002291573 ISSN: 0006-291X cité dans la demande**
- **KIM K B ET AL: "Proteasome Inhibition by the Natural Products Epoxomicin and Dihydroeponemycin: Insights into Specificity and Potency" BIOORGANIC & MEDICINAL CHEMISTRY LETTERS, OXFORD, GB, vol. 9, no. 23, 6 décembre 1999 (1999-12-06), pages 3335-3340, XP004183734 ISSN: 0960-894X**
- **SPATOLA A F: "Peptide backbone modifications: a structure-activity analysis of peptides containing amide bond surrogates" 1983, CHEMISTRY AND BIOCHEMISTRY OF AMINO ACIDS, PEPTIDES, AND PROTEINS, PAGE(S) 267-357 , XP002086355 cité dans la demande le document en entier**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 699 815 B1

Printed by Jouve, 75001 PARIS (FR)

## Description

La présente invention a pour objet de nouvelles molécules et leur utilisation pour moduler l'activité du protéasome. Elle a également pour objet les compositions pharmaceutiques et cosmétiques les comprenant et l'utilisation de ces molécules pour la prévention et/ou le traitement de pathologies et des désordres liés au protéasome.

Le protéasome est une enzyme protéolytique essentielle du cytoplasme et du noyau des cellules eukaryotes. Il est impliqué dans la dégradation de la plupart des protéines intracellulaires et participe à la transformation des antigènes présentés par la plupart des molécules MHC-1.

Au moins cinq types d'activités protéolytiques ont été identifiés dont trois principales : une activité de type chymotrypsine (CT-L), une activité de type trypsine (T-L) et une activité peptidase post-acide. Le site catalytique de type peptidase post-acide coupe préférentiellement les séquences peptidiques comportant un acide glutamique en position P1 ; le site catalytique de type trypsine coupe préférentiellement mais non exclusivement les séquences peptidiques comportant un acide aminé basique (arginine, lysine) en position P1 ; le site catalytique de type chymotrypsine coupe préférentiellement mais non exclusivement les séquences peptidiques comportant un acide aminé hydrophobe, tel qu'une leucine en position P1.

La structure du protéasome est celle d'un complexe protéique 26S (2,4 MDa) comportant un complexe catalytiquement actif dénommé 20S, dont l'activité est régulée par des complexes régulateurs.

Le protéasome hydrolyse les protéines en fragments de 3 à 25 résidus avec une moyenne de 7 à 8 résidus.

Le particule catalytique du protéasome, 20S, peut être sous deux états distincts, l'un activé et l'autre non activé.

Le protéasome est un élément indispensable à la protéolyse intracellulaire, qu'elle soit dépendante ou non de l'ubiquitine (Eytan et al., Proc. Natl. Acad. Sci. USA 86:7751-7755 (1989) ; Reichsteiner et al. J. Biol. Chem. 268: 6065-6068 (1993)). Ces mécanismes sont impliqués dans la dégradation des cyclines et d'autres protéines à courte durée de vie ainsi qu'à longue durée de vie. Les oncogènes (Glotzer et al., Nature 349:132-138 (1991) ; Ciechanover et al., Proc. Natl. Acad. Sci. USA 88, 139-143 (1991)) et l'ornithine décarboxylase (Murakami et al., Nature, 360:597-599 (1992)) constituent des exemples de protéines dégradées. Ces données suggèrent fortement que le protéasome joue un rôle important dans la régulation de la croissance cellulaire et dans la mitose.

Le protéasome joue également un rôle-clé dans la présentation des peptides antigéniques aux cellules du système immunitaire, donc dans la surveillance dirigée contre les virus et le cancer (Brown et al., Nature, 355:355-360 (1991)).

Le rôle joué par le protéasome dans la dégradation des protéines suggère que son inhibition puisse permettre d'agir sur des pathologies comme le cancer, les maladies auto-immunes, le SIDA, les maladies inflammatoires, les maladies cardiaques, le rejet de greffe, la fonte musculaire (M. Reboud-Ravaux, Progress in Molecular and Subcellular Biology, vol. 29, Springer Verlag, 2002, p. 109-125 ; Kisselev et al., Chemistry & Biology, 8, 739-758 (2001)).

Par ailleurs, on sait que l'activation du protéasome devrait permettre d'agir sur les mécanismes de protéolyse intracellulaire dans le sens d'une accélération de ces mécanismes qui peut être souhaitée par exemple lorsque l'on constate une accumulation de protéines oxydées. Dans ce contexte une molécule activatrice du protéasome devrait permettre d'éliminer les protéines oxydées et constituer un traitement et/ou une méthode d'inhibition de l'apparition des signes du vieillissement, notamment du vieillissement cutané. Des molécules activatrices du protéasomes ont été décrites notamment par : Kisselev et al., J. Biol. Chem, 277, 22260-22270 (2002) ; Wilk et al., Mol. Biol. Rep., 24, 119-124 (1997) ; Ruiz De Mena et al., Biochem. J., 296, 93-97 (1993) ; Arribas et al., J. Biol. Chem., 265, 13969-13973 (1990).

L'accumulation de protéines est également observée dans le contexte de la maladie d'Alzheimer et de la maladie de Parkinson. Une activation du protéasome pourrait permettre d'activer le processus de dégradation des protéines dans le traitement de ces pathologies. Des composés de ce type sont décrits dans les documents US-5,847,076 et JP-2002029996.

Un inhibiteur du protéasome existe déjà sur le marché : le Velcade ® est utilisé pour le traitement du myélome multiple. Le Velcade ® se lie de façon covalente aux sites actifs du protéasome et bloque ainsi leur activité. Il empêche ainsi le protéasome d'effectuer la dégradation des protéines et bloque notamment le processus d'apoptose et de mort cellulaire (Richardson et al., Cancer Control, 10, 361-366 (2003)).

Toutefois ce mécanisme d'action, extrêmement efficace, se révèle aussi être toxique pour l'organisme et a pour conséquence des effets secondaires importants. Le problème se pose donc de trouver des inhibiteurs du protéasome qui soient moins drastiques dans leur mécanisme d'action.

La difficulté pour concevoir des inhibiteurs du protéasome est d'autant plus grande que le protéasome montre une spécificité médiocre dans le choix de ses substrats et dans le schéma de clivage qu'il adopte.

Dans l'article « *Proteasome Inhibition by Natural Products Epoxomicin and Dihydroeponemycin : Insights into Specificity and Potency* » *Kim et al.* décrivent des composés inhibiteurs et leur spécificité de liaison aux sites actifs du protéasome, en particulier le protéasome 20S. Spécifiquement, ces composés sont des chimères biotinylés d'epoxomicine et de dihydroeponemycine

La demande brevet internationale WO 98/338 12 A décrit des inhibiteurs de la protéase des cellules adipeuses

et leur utilisation dans le traitement des désordres inflammatoires dus à l'activité tryptase des cellules adipeuses. En particulier, sont mis en évidence dans cette demande des inhibiteurs *in vitro* ou *in vivo* du complexe tryptase-6 des cellules adipeuses. Les inhibiteurs de protéase décrits sont des inhibiteurs irréversibles et compétitifs. Ils se lient au site de liaison de la protéase pour le substrat de manière covalente.

L'un des problèmes que vise à résoudre l'invention a été la mise au point de molécules se liant de manière non covalente aux sites actifs du protéasome et/ou aux sites régulateurs du protéasome.

On connaît, par le document Bioorganic and Medicinal Chemistry, 11 (2003), 4881-4889, des pseudopeptides dérivés de la séquence Ac-Leu-Leu-Norleucinal. Ces composés sont des inhibiteurs potentiels du protéasome. Toutefois, leur activité sur le protéasome n'est pas quantifiée.

On a également cherché à mettre au point des molécules de petite taille dont la synthèse soit simple et reproductible afin d'être industrialisable. On a en outre souhaité obtenir des molécules stables, y compris pour une administration orale.

On connaît, notamment par le document Papapostolou et al., BBRC, 295 (2002) 1090-1095, des peptides de petite taille (5 à 6 acides aminés) qui se lient de façon non covalente au protéasome et qui ont une activité modulatrice (activatrice pour les uns, inhibitrice pour les autres) des fonctions du protéasome.

Toutefois, l'affinité de ces molécules pour leur cible peut encore être améliorée et leur stabilité dans des conditions d'administration à un organisme humain laissé à désirer.

Les inventeurs se sont donc fixé pour objectifs la conception et la synthèse de molécules nouvelles qui n'aient pas les inconvénients des molécules de l'art antérieur.

Cet objectif a été atteint par les molécules de l'invention qui répondent à la formule générale (I) ci-dessous, et leurs sels pharmaceutiquement acceptables :

$$(X_0)_{x0}\text{-}(X_1)_{x1}\text{-}(X_2)_{x2}\text{-}X_3\text{-}(X_4)_{x4}\text{-}X_5\text{-}X_6\text{-}(X_7)_{x7}\text{-}(X_8)_{x8}\text{-}(X_9)_{x9} \quad \text{(I)}$$

dans laquelle $x_0$, $x_1$, $x_2$, $x_4$, $x_7$, $x_8$ et $x_9$ représentent chacun, de façon indépendante, un entier égal à 0 ou à 1 ;
$X_0$ représente un groupement choisi parmi ceux répondant à la formule (II) :

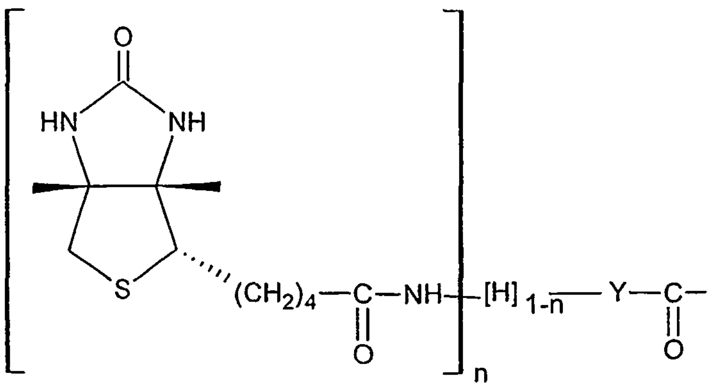

(II)

dans laquelle Y représente un groupement alkyle en $C_1$-$C_{24}$, linéaire ramifié ou cyclique, saturé ou insaturé, n représente un entier choisi parmi 0 et 1.

Suivant les cas, l'on a:
- n = 1 et $X_0$ représente un groupement biotinyle greffé sur une chaîne amino-acyle.
- n = 0 et $X_0$ représente une chaîne acyle HY-CO-.

$X_1$ et $X_3$ représentent chacun un acide aminé naturel ou synthétique de configuration L ou D comportant chacun au moins une fonction hydroxyle sur sa chaîne latérale. $X_1$ et $X_3$, identiques ou différents peuvent être choisis par exemple parmi la thréonine et la sérine ;

$X_2$ représente un acide aminé naturel ou synthétique de configuration L ou D qui peut être choisi parmi ceux comportant une chaîne latérale alkyle, comme par exemple la valine, la leucine, l'isoleucine ;

$X_4$ représente un acide aminé naturel ou synthétique de configuration L ou D qui peut être choisi parmi ceux comportant une chaîne latérale aromatique comme par exemple la phénylalanine, le tryptophane et la tyrosine ; $X_4$ peut également être un acide aminé aromatique comportant un groupe réactionnel photoactivable comme la *para*-benzoyl phénylalanine ;

$X_5$ représente un acide aminé de configuration L ou D sélectionné parmi : les acides aminés chargés positivement tels que la lysine, l'arginine, l'histidine ; les acides aminés chargés négativement, tels que l'acide aspartique, l'acide glutamique ; les acides aminés porteurs d'une fonction amide, tels que l'asparagine et la glutamine ;
$X_6$ représente un acide aminé de configuration L ou D qui peut être choisi parmi la tyrosine, la phénylalanine, la leucine, l'isoleucine, l'alanine ; $X_6$ peut également être un acide aminé aromatique comportant un groupe réactionnel photoactivable comme la *para*-benzoyl phénylalanine ; $X_6$ peut encore être le lysine ;
$X_7$ représente un acide aminé de configuration L ou D qui peut être choisi parmi la glycine l'alanine, la leucine, la valine, l'asparagine et l'arginine ;
$X_8$ représente un acide aminé de configuration L ou D qui peut être choisi parmi la proline, la valine, l'isoleucine, l'acide aspartique ;
$X_9$ représente un acide aminé de configuration L ou D qui peut être choisi parmi la sérine, l'alanine, la lysine, l'arginine, le tryptophane ;
la liaison entre deux acides aminés successif $X_i$-$X_{i+1}$, désignée $q_{i}$-$_{i+1}$, i = 1...8, peut être une liaison peptidique

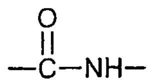

ou une liaison pseudopeptidique choisie notamment dans la liste suivante :

| | |
|---|---|
| ester | CO-O |
| thioester | CO-S |
| cétométhylène | $CO$-$CH_2$ |
| N-méthylamide | CO-N(Me) |
| amide inversée | NH-CO |
| Z/E vinylène | CH=CH |
| éthylène | CH2-$CH_2$ |
| méthylènethio | CH2-S |
| méthylèneoxy | $CH_2$-O |
| thioamide | CS-NH |
| méthylène amino | $CH_2$-NH |
| cétométhylène amino | CO-$CH_2$-NH |
| hydrazino | CO-NH-NH |
| carbonylhydrazone | CO-NH-N= |
| N-amino | CO-N($NH_2$) |

- les acides aminés énoncés ci-dessus $X_i$, i = 1,... 9 étant susceptibles de comporter une modification de leur carbone α, désigné Ci, i = 1, ... 9 et porteur de la chaîne latérale R de l'acide aminé, modification consistant dans le remplacement de :

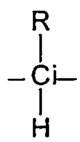

par un groupement choisi parmi :

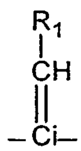 , 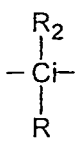 et 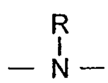

les groupements R et CH-$R_1$ représentant la chaîne latérale de l'acide aminé et $R_2$ représentant un groupement alkyle en $C_1$-$C_6$ ; éventuellement R-$R_2$ peuvent constituer un cycle,
les pseudopeptides de l'invention répondant en outre aux conditions suivantes :

- $x_0$ est égal à 1
  ou
- l'une des liaisons $q_{i-i+1}$, i=1,...8, est une liaison pseudopeptidique
  ou
- l'un des $C_i$, i=1,...9, comporte l'une des modifications énoncées ci-dessus.

En effet, comme cela est illustré dans la partie expérimentale, les molécules de formule (I), qui comportent au moins un groupement non peptidique, ont en commun la propriété de se lier de façon non covalente aux sites actifs et/ou aux sites régulateurs du protéasome. En particulier, elles ont la propriété de se lier aux sites actifs et/ou aux sites régulateurs de l'activité CT-L (chymotrypsine-like) du protéasome
Certaines de ces molécules ont une activité inhibitrice du protéasome, d'autres sont activatrices du protéasome. Certaines molécules, comportant un groupement photoactivable *para*-benzoyl-phénylalanine, peuvent, par l'application d'un traitement photochimique, se fixer de façon covalente au protéasome.
On a constaté que dans des tests pratiqués *in vitro* les molécules de l'invention étaient dotées d'une plus grande affinité pour le protéasome que les molécules de l'art antérieur décrites dans Papapostolou et al., BBRC, 295 (2002) 1090-1095, qui ont une structure strictement peptidique.
En outre, leur caractère non strictement peptidique (la présence de liaison(s) non peptidique(s) et/ou de certains acides aminés modifiés) permet de prévoir une moindre efficacité des protéases sur la dégradation de ces molécules et donc une meilleure résistance à la protéolyse dans des conditions d'administration *in vivo.*
Outre les caractéristiques pseudopeptidiques énoncées ci-dessus, les acides aminés utilisés pour la préparation des molécules de formule (I) peuvent être des acides aminés naturels, sous forme d'énantiomère L. Toutefois, on peut prévoir d'utiliser leurs analogues D ou leurs analogues β-aminés, γ-aminés ou ω-aminés.
Les molécules de l'invention comportent au moins l'une des caractéristiques suivantes :

- chaîne biotinyle ou acyle en extrémité N-terminale,
- ou liaison peptidique modifiée,
- ou présence d'un acide aminé comportant un carbone α modifié, chacune de ces modifications consistant en une variante par rapport à un enchaînement peptidique simple :

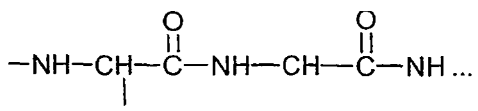

Toutefois, les molécules de l'invention peuvent comporter plus d'une modification par rapport à un enchaînement peptidique simple, comme par exemple :

- un groupement acyle en extrémité N-terminale et une ou plusieurs liaisons pseudopeptidiques.
- un groupement biotinyle en extrémité N-terminale et un groupement *para*-benzoyl-phénylalanine dans la chaîne peptidique,
- une liaison pseudopeptidique et un acide aminé comportant un carbone α modifié,
- un groupement acyle N-terminal et un acide β ou γ-aminé.

Lorsque $x_0$=1, la chaîne acyle -Y-CO- peut être linéaire ramifiée ou cyclique, saturée ou insaturée. De préférence c'est une chaîne linéaire que l'on représente par la formule $-C_pH_{2p}-CO-$, p étant un entier allant de 1 à 23.
De préférence, l'un au moins des entiers $x_0$, $x_1$, $x_2$, $x_4$, $x_7$, $x_8$ et $x_9$ est égal à 1.
Parmi les molécules répondant à la formule (I), on préfère celles comprenant 4 à 8 acides aminés, préférentiellement 5 à 7 acides aminés, encore plus préférentiellement celles comportant 6 acides aminés.
Dans le cas où $x_0$=1 :

- lorsque n = 1, de préférence Y comporte 1 à 8 atomes de carbone, par exemple Y représente $-C_pH_{2p}-$ et p peut être 1, 2, 3, 4, 5, 6, 7 ou 8
- lorsque n = 0, de préférence Y comporte de 5 à 23 atomes de carbone par exemple Y représente $-C_pH_{2p}-$ et p peut être un entier allant de 5 à 23.

De préférence, l'un au moins de $X_1$ et de $X_3$ représente la thréonine. Encore plus préférentiellement $X_1$ et $X_3$ représentent tous deux la thréonine.

De préférence $X_2$ est choisi parmi l'isoleucine et la valine.

De préférence $X_4$ est choisi parmi la phénylalanine, la tyrosine et la *para*-benzoyl-phénylalanine.

De préférence au moins 2 des entiers $x_0$, $x_1$, $x_2$, $x_4$, $x_7$, $x_8$ et $x_9$ sont égaux à 1, encore plus préférentiellement 3 au moins de ces entiers sont égaux à 1,

Parmi les molécules répondant à la formule (I), une séquence préférée est celle répondant à la formule (Ia) :

$X_0$-$X_1$-$X_2$-$X_3$-$X_4$-$X_5$-$X_6$ (Ia)

dans laquelle $X_0$, $X_1$, $X_2$, $X_3$, $X_4$, $X_5$, $X_6$ ont la même définition que ci-dessus, les liaisons $q_{i,\ i+1}$ entre les acides aminés $X_i$ et $X_{i+1}$, i = 1...5, étant des liaisons peptidiques ou pseudo peptidiques.

Selon une première variante préférée de la molécule (Ia) $X_0$ représente :

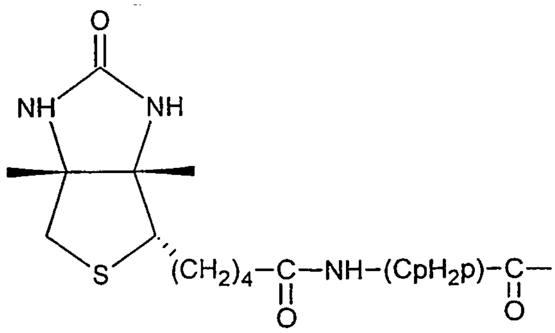

avec p allant de 1 à 8, de préférence de 2 à 6,
et $X_4$ représente un groupement *para*-benzoyl-phénylalanine.

Selon une seconde variante préférée de la molécule (Ia), $X_0$ représente un groupement acyle :

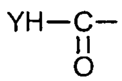

dans lequel Y représente un groupement alkyle en $C_3$-$C_{23}$.
Encore plus préférentiellement $X_0$ représente un groupement : $(C_pH_{2p+1})$

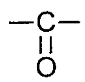

avec p allant de 3 à 23, de préférence de 5 à 19.

Parmi les molécules répondant à la formule (I), une autre séquence préférée est celle répondant à la formule (Ib) :

$X_3$-$X_5$-$X_6$-$X_7$-$X_8$-$X_9$ (Ib)

dans laquelle $X_3$, $X_5$, $X_6$, $X_7$, $X_8$ et $X_9$ ont la même définition que ci-dessus,

- l'une au moins des liaisons entre deux acides aminés successifs étant une liaison pseudopeptidique
ou
- l'un des carbones α de l'un des acides aminés étant un carbone α modifié.

Selon l'invention, le terme sels se rapporte à la fois aux sels d'amine d'une fonction carboxyle de la chaîne peptidique aussi bien qu'aux sels acides d'addition à un groupe amine de cette même chaîne polypeptidique. Les sels d'une fonction carboxyle peuvent être formés avec une base inorganique ou organique. Les sels inorganiques incluent

par exemple les sels de métaux alcalins tels que les sels de sodium, de potassium et de lithium ; les sels alcalino-terreux tels que par exemple les sels de calcium, de barium et de magnésium ; les sels d'ammonium, les sels ferreux, ferriques, les sels de zinc, de manganèse, d'aluminium, de magnésium. Les sels avec des amines organiques incluent ceux formés par exemple avec la triméthylamine, la triéthylamine, la tri(n-propyl)amine, la dicyclohexylamine, la triéthanolamine, l'arginine, la lysine, l'histidine, l'éthylènediamine, la glucosamine, la méthylglucamine, les purines, les pipérazines, les pipéridines, la caféine, la procaine.

Les sels d'addition acides incluent, par exemple, les sels avec des acides minéraux tels que par exemple l'acide chlorhydrique, l'acide bromhydrique, l'acide sulfurique, l'acide phosphorique, l'acide nitrique ; les sels avec des acides organiques tels que par exemple, l'acide acétique, l'acide trifluoroacétique, l'acide oxalique, l'acide tartrique, l'acide succinique, l'acide maléique, l'acide fumarique, l'acide gluconique, l'acide citrique, l'acide malique, l'acide ascorbique, l'acide benzoïque.

Parmi les molécules préférées de l'invention, on peut citer :

$CH_3$-($C_nH_{2n}$)-CO-TVTYDY avec n=4,6,8,10,12,14,16,18
$CH_3$-($C_nH_{2n}$)-CO-TISYDY avec n=4,6,8,10,12,14,16,18
$CH_3$-($C_nH_{2n}$)-CO-TVSYKF avec n=4,6,8,10,12,14,16,18
$CH_3$-($C_nH_{2n}$)-CO-TITFDY avec n=4,6,8,10,12,14,16,18
$CH_3$-($C_nH_{2n}$)-CO-TITYKF avec n=4,6,8,10,12,14,16,18
$CH_3$-($C_nH_{2n}$)-CO-TITYEY avec n=4,6,8,10,12,14,16,18
$CH_3$-($C_nH_{2n}$)-CO-TITYDF avec n=4,6,8,10,12,14,16,18
$CH_3$-($C_nH_{2n}$)-CO-TVTYKL avec n=4,6,8,10,12,14,16,18
$CH_3$-($C_nH_{2n}$)-CO-TVTYKY avec n=4,6,8,10,12,14,16,18
$CH_3$-($C_nH_{2n}$)-CO-TVTFKF avec n=4,6,8,10,12,14,16,18
$CH_3$-($C_nH_{2n}$)-CO-TITYDL avec n=4,6,8,10,12,14,16,18
$CH_3$-($C_nH_{2n}$)-CO-TITFDY avec n=4,6,8,10,12,14,16,18
$CH_3$-($C_nH_{2n}$)-CO-TVTFKF avec n=4,6,8,10,12,14,16,18
$CH_3$-($C_nH_{2n}$)-CO-TVTYKF avec n=4,6,8,10,12,14,16,18
Biot-Ava-TVT-Bpa-KF
Biot-Ava-TVT-Bpa-KY
Biot-Ava-TVT-Bpa-KL
Biot-Ava-TVT-Bpa-DF
Biot-Ava-TVT-Bpa-DY
Biot-Ava-TVT-Bpa-DL
Biot-Ava-TIT-Bpa-KF
Biot-Ava-TIT-Bpa-KY
Biot-Ava-TIT-Bpa-KL
Biot-Ava-TIT-Bpa-DF
Biot-Ava-TIT-Bpa-DY
Biot-Ava-TIT-Bpa-DL
Biot-Ava-TVT-Bpa-EF
Biot-Ava-TVT-Bpa-EY
Biot-Ava-TVT-Bpa-EL
Biot-Ava-TIT-Bpa-EF
Biot-Ava-TIT-Bpa-EY
Biot-Ava-TIT-Bpa-EL
Biot-Ava-TVT-Bpa-NF
Biot-Ava-TVT-Bpa-NY
Biot-Ava-TVT-Bpa-NL
Biot-Ava-TIT-Bpa-NF
Biot-Ava-TIT-Bpa-NY
Biot-Ava-TIT-Bpa-NL

TNL*GPS, ou encore SEK*RVW, TRA*LVR, SNL*NDA et THI*VIK, dans lesquelles * représente :

- une liaison choisie parmi les liaisons ester, thioester, cétométhylène, cétométhylèneamino, N-méthylamide, amide inversée, Z/E vinylène, éthylène, méthylènethio, méthylèneoxy, thioamide, méthylèneamide, hydrazino, carbonylhydrazone et N-amino,

  ou

- la présence d'un aza-acide aminé en substitution de l'un des acides aminés adjacents à *.

Biot représente un groupement biotinyle, Ava représente un groupement acide δ amino valérique, Bpa représente un groupement *para*-benzoyl-phénylalanine.

Selon l'invention, on peut également prévoir que les molécules décrites ci-dessus soient couplées sur leur extrémité C-terminale et/ou, lorsque cela est possible, sur leur extrémité N-terminale, avec une autre molécule qui favorise la biodisponibilité de la molécule de l'invention. On peut notamment citer à cet effet les peptides qui favorisent la pénétration dans la cellule et qui sont décrits notamment dans : ROJAS et al, Nat. Biotechnol., 16, 370-375 (1998) ; FUTAKI et al, J. Biol. Chem., 276, 5836-5840 (2001) ; MORRIS et al, Nat. Biotechnol., 19, 1173-1176 (2001). On peut citer également le produit dénommé pénétratine et les vecteurs peptidiques commercialisés par la société Diatos.

Les molécules de l'invention peuvent être préparées selon des techniques bien connues de l'homme du métier comme la synthèse peptidique et la synthèse de pseudopeptides. Ces techniques de synthèse sont illustrées dans la partie expérimentale. Pour la synthèse de pseudopeptides, on peut se reporter par exemple à : SPATOLA, Vega Data, Vol.1, issue 3 (1983) ; SPATOLA, Chemistry and Biochemistry of Amino Acids Peptides and Proteins, Weinstein, ed., Marcel Dekker, New York, p.267 (1983). MORLEY.J.-S., Trends Pharm. Sci., 463-468 (1980) ; HUDSON et al, Int. J. Pept. Prot. Res. 14, 177-185 (1979); SPATOLA et al, Life Sci., 38, 1243-1249 (1986); Hann, J.Chem. Soc. Perkin Trans.I 307-314 (1982) ; ALMQUIST et al, J. Med. Chem., 23, 1392-1398 (1980) ; JENNINGS-WHITE et al, EP-45665 ; HOLLADAY et al, Tetrahedron Lett. 24, 4401-4404 (1983). HRUBY et al, Life Sci. 31, 189-199 (1982).

Un peptide modifié selon l'invention peut également être obtenu par expression d'un peptide à partir d'une molécule d'acide nucléique recombinant puis modification (greffe d'un groupement *para*-benzoyle sur un résidu phénylalanine, greffe d'un groupement biotinylaminoacyle, d'un groupement acyle).

Les molécules de l'invention peuvent être utilisées pour moduler l'activité du protéasome ; ces utilisations constituent un autre objet de l'invention :

L'invention a notamment pour objet l'utilisation d'une molécule décrite ci-dessus, pour la préparation d'un médicament destiné à la prévention et/ou au traitement d'une pathologie impliquant le protéasome et en particulier son activité de type chymotrypsine (CT-L).

Certaines de ces molécules ont des propriétés inhibitrices de l'activité du protéasome, et à ce titre elles peuvent être utilisées pour préparer un médicament destiné à la prévention et/ou au traitement d'une pathologie sélectionnée parmi : les cancers impliquant des tumeurs hématologiques comme le myélome multiple, les leucémies, les lymphomes, les sarcomes: RICHARSON et al, Cancer Control, 10, 361-366 (2003); ADAMS,Drug Discovery Today, 8, 307-311 ; ou des tumeurs solides de la rate, du sein, du colon, du rein, du tractus othorhinolaryngopharingé, du poumon, de l'ovaire, de la prostate, du pancréas, de la peau : LENZ, Cancer Treatment Reviews, 29, 41-48 (2003) ; les maladies inflammatoires telles que par exemple la maladie de Crohn et l'asthme : ELLIOT et al, J. Allergy Clin. Immunol. 104, 294-300 (1999) ; ELLIOT et al, Journal of Molecular Medecine, 81, 235-245 (2003) ; la fonte musculaire : LECKER et al, J. Nutr. 129, 227S-237S (1999) ; le sida : SCHUBERT, Proc. Natl. Acad. Sci. USA, 97, 1357-1362 (2000); les maladies autoimmunes comme par exemple la polyarthrite rhumatoïde et le lupus érythémateux aigu disséminé : Schwartz et al, J. Immunol, 164, 6114-6157 (2000); les pathologies cardiaques comme par exemple les myocardites et les conséquences des processus ischémiques que ce soit au niveau myocardique ou cérébral ou pulmonaire: CAMPBELL et al, J. Mol. Cell Cardiol. 31, 467-476 ; les accidents vasculaires cérébraux : ZHANG et al, Curr. Drug Targets Inflamm. Allergy 1, 151-156 (2002), DI NAPOLI et al, Current Opinion Invest. Drugs, 4, 303-341 (2003), le rejet d'allogreffe ; les traumatismes, les brûlures, la régénération cornéenne : STRAMER et al, Invest. Ophthalmol. Vis. Sci. 42, 1698-1706 (2001).

Certaines de ces molécules ont une activité stimulatrice de l'action du protéasome et à ce titre, elles peuvent être utilisées pour la préparation d'un médicament destiné à la prévention ou au traitement de certaines pathologies liées au vieillissement, comme par exemple la maladie d'Alzheimer : TSUJI et SHIMOHAMA, dans M. Reboud-Ravaux, Progress in Molecular and Subcellular Biology, vol. 29, Springer Verlag, 2002, p. 42-60, et la maladie Parkinson : SIDELL et al, J. Neur. Chem., 79, 510-521 (2001).

Les molécules stimulatrices de l'action du protéasome peuvent également être utilisées en cosmétique ou en dermatologie, pour la préparation de compositions destinées à retarder et/ou à traiter les effets du vieillissement cutané chronologique ou actinique (photovieillissement) : FISHER et al, Photochem. Photobiol. 69, 154-157 (1999). Les protéines oxydées s'accumulent dans les fibroblastes âgés de la peau alors que le protéasome, responsable de la dégradation des protéines oxydées, voit son activité diminuée : GRUNE, Hautartz, 54, 818-821 (2003); LY et al, Science, 287, 2486-2492 (2000). Un procédé de prévention ou de traitement cosmétique de l'apparition des effets du vieillissement cutané physiologique et/ou actinique comportant l'application d'une molécule selon l'invention, dans un support cosmétiquement acceptable est décrit. Parmi les symptômes du vieillissement cutané, on peut citer notamment l'apparition des rides, un teint terne, le relâchement de la peau, la perte d'élasticité.

Les molécules de l'invention peuvent être utilisées seules ou en association avec un ou plusieurs autres principes actifs, aussi bien dans le domaine thérapeutique (traitement anti-cancéreux, polythérapie anti-sida...) que dans le domaine cosmétique. Elles peuvent également être utilisées conjointement à un traitement de radiothérapie.

Les molécules de l'invention peuvent également être utilisées pour la préparation d'un médicament destiné à la radiosensibilisation d'une tumeur.

L'invention a également pour objet un médicament comportant des molécules de l'invention dans un support pharmaceutiquement acceptable.

Le choix du support et des adjuvants sera guidé par le mode d'administration qui sera adapté en fonction du type de pathologie à traiter. On peut prévoir une administration par voie orale ou parentérale.

La quantité de molécule de formule (I) à administrer à l'homme, ou éventuellement à l'animal, dépend de l'activité propre à cette molécule, activité qui peut être mesurée par des moyens qui seront exposés dans les exemples. Elle dépend également du degré de gravité de la pathologie à traiter.

L'invention a en outre pour objet une composition cosmétique et/ou dermatologique comprenant une molécule de l'invention dans un support cosmétiquement et/ou dermatologiquement acceptable. Un tel support peut être par exemple une crème, une lotion, un lait, un onguent, un shampooing.

## PARTIE EXPERIMENTALE

### A-SYNTHESE DES MOLECULES

#### 1- Lipopeptides

17 lipopeptides ont été synthétisés, leur structure est donnée dans le tableau I :

Tableau I : Séquences synthétisées

| Séquences | TITFDY | TVTFKF | TVTYKF |
|---|---|---|---|
| Chaîne aliphatique | $CH_3-(CH_2)_4-CO-$ | $CH_3-(CH_2)_4-CO-$ | $CH_3-(CH_2)_4-CO-$ |
| | $CH_3-(CH_2)_6-CO-$ | $CH_3-(CH_2)_6-CO-$ | $CH_3-(CH_2)_6-CO-$ |
| | $CH_3-(CH_2)_8-CO-$ | $CH_3-(CH_2)_8-CO-$ | $CH_3-(CH_2)_8-CO-$ |
| | | $CH_3-(CH_2)_{10}-CO-$ | $CH_3-(CH_2)_{10}-CO-$ |
| | | $CH_3-(CH_2)_{12}-CO-$ | $CH_3-(CH_2)_{12}-CO-$ |
| | | $CH_3-(CH_2)_{14}-CO-$ | $CH_3-(CH_2)_{14}-CO-$ |
| | | $CH_3-(CH_2)_{16}-CO-$ | $CH_3-(CH_2)_{16}-CO-$ |

Les lipopeptides sont synthétisés sur un synthétiseur semi-automatique (CNRS, IBMC, Strasbourg, France) (1. Neimark, J., and Briand, J. P. (1993) Pept. Res. 6, 219-228) à partir de résines Fmoc-Leu(tBu)-Wang PS, Fmoc-Lys (Boc)-Wang PS et Fmoc-Tyr(tBu)-Wang PS (Senn Chemicals International (Dielsdorf, Suisse). La stratégie utilisée est un protocole classique Fmoc/tBu. L'élongation de la chaîne peptidique est réalisée par couplage et déprotection successifs des Fmoc-aminoacides (3éq. par rapport à la substitution de la résine. Les acides aminés utilisés (Neosystem (Strasbourg, France) ou Senn Chemicals International (Gentilly, France)) sont : Fmoc-Thr(tBu)-OH, Fmoc-Tyr(tBu)-OH, Fmoc-Ser(tBu)-OH, Fmoc-Asp(OtBu)-OH, Fmoc-Gln(OtBu)-OH et Fmoc-Lys(Boc)-OH. Les catalyseurs de couplage sont le tétrafluoroborate de 2-(1H-benzotriazole-1-yl)-1,1,3,3-tetraméthyluronium (TBTU), (3éq.), le 1-hydroxybenzotriazole (HOBt) (3éq.) et la diisopropyléthylamine (DIEA) (9éq.) dans la *N,N*-diméthylformamide (DMF).

L'avancement de chaque étape est contrôlé par un test colorimétrique à l'acide 2,4,6-trinitrobenzènesulfonique. La déprotection N-terminale du groupe Fmoc est réalisée par une solution de pipéridine à 20% dans la DMF.

La chaîne lipidique est couplée à partir des chlorures d'acides (3éq.) en présence de DIEA (9éq.).

Les peptides sont clivés de la résine pendant 2 heures par un mélange de 10 mL de TFA, 0,750 g de phénol, 0,25 mL d'EDT, 0,5 mL de thioanisole et 0,5 mL d'eau désionisée. Ce mélange est initialement additionné à la résine-peptide à 0°C mais le clivage se déroule à température ambiante. Les peptides précipitent par ajout d'$Et_2O$ glacé et la résine est filtrée. Le peptide resté sur le fritté est dissous au-dessus d'un ballon plein d'$Et_2O$ glacé à l'aide de TFA. Il est ensuite concentré et lyophilisé.

Les peptides sont purifiés par chromatographie liquide haute performance (HPLC) réalisée sur une chaîne Hitachi-Merck équipée d'une pompe L6200 couplée à un détecteur Jasco 875 UV. La colonne préparative utilisée est une Macherey-Nagel Nucleosil 300-7 C4 (250 X 10 mm i.d.). L'éluant est composé d'une solution A de 0,1 % en volume de TFA (sequencing grade, Sigma) dans l'eau Ultrapure et d'une solution B de 0,08 % de TFA et de 20 % d'eau dans l'acétonitrile (Carlo Erba). Le peptide est élué par un gradient de 20% de B dans A jusque 50% sur 30 minutes à 4mL/minute. Le peptide est collecté manuellement. Après évaporation des solvants, le peptide purifié est lyophilisé avant

d'être caractérisé par spectrométrie de masse et RMN.

## 2- Pseudopeptides

### 2.1 Peptides réduits

**a- Protocole opératoire pour la préparation du Fmoc-leucinal**

(Douat C., Heitz A., Martinez J., Fehrentz J.A., *Tetrahedron Lett.*, **2000**, 41, 37-40) : Ce protocole opératoire est résumé par le schéma 1, ci-dessous :

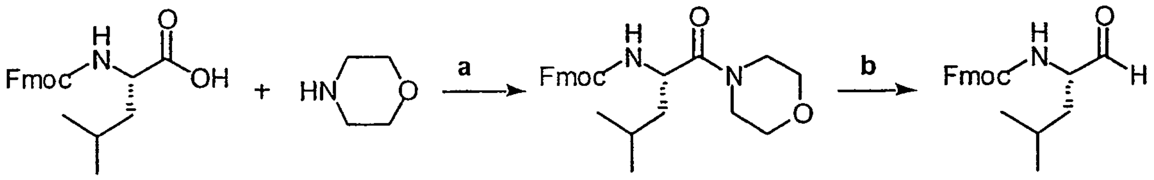


**1** **2** **3** **4**

**a**: IBCF, NMM. **b**: $LiAlH_4$

Schéma 1 : Synthèse du Fmoc-leucinal

**b- Synthèse de Fmoc-Leu-N($CH_2$-$CH_2$)$_2$O :**

Fmoc-Leu-H a été synthétisé comme décrit par Douat et coll. (§a ci-dessus) 4,81 mmoles (0,53 mL) de N-méthylmorpholine et 4,81 mmoles (0,62 mL) de chloroformiate d'isobutyle (IBCF) sont additionnées goutte-à-goutte à -15°C à une solution de Fmoc-Leu-OH (4,81 mmoles, 1,7 g) dans du THF anhydre (10 mL) sous courant d'azote La solution est agitée par un barreau aimanté couplé à une plaque d'agitation magnétique. Le milieu réactionnel est agité pendant 15 minutes, filtré et lavé deux fois par du THF anhydre. Toujours sous azote, 4,81 mmoles (0,42 mL) de morpholine sont additionnées goutte-à-goutte et le mélange est agité à température ambiante pendant 1 heure. Le solvant est évaporé sous vide sur un évaporateur rotatif et le résidu repris par 50 mL d'acétate d'éthyle, lavé par une solution aqueuse à 5% de $KHSO_4$ (15 mL), une solution aqueuse à 5% de $KHCO_3$ (15 mL), puis de l'eau désionisée (2 X 10 mL). La phase organique est séchée sur $MgSO_4$ et évaporée sous vide sur un évaporateur rotatif. Le produit brut (1,88 g) est purifié par chromatographie sur colonne de silice avec pour éluant un mélange acétate d'éthyle : hexane 70 : 30 (Rf = 0,40). Le produit se présente sous la forme d'une mousse blanche (rendement 69%, 1,4 g, 3,31 mmol).

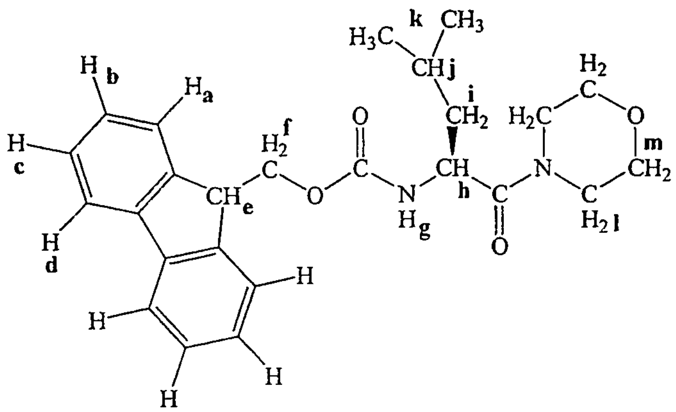

RMN $^1$H (300 MHz, $CDCl_3$) : 0,94 ppm (3H, d, $J_{k-j}$ = 6,5 Hz, $H_k$) ; 0,99 ppm (3H, d, $J_{k-j}$ = 6,5 Hz, $H_k$) ; 1,54 ppm (2H, m, $H_i$) ; 1,69 ppm (1H, m, $H_j$) ; 3,47 ppm (4H, m, $H_l$) ; 3,66 ppm (4H, m, $H_m$) ; 4,22 ppm (1H, t, $J_{e-f}$ = 6,7 Hz, $H_e$) ; 4,37 ppm

(2H, m, $H_f$) ; 4,70 ppm (1H, m, $H_h$) ; 5,57 ppm (1H, d, $J_{g-h}$ = 8,8 Hz, $H_g$) ; 7,31 ppm (2H, m, $H_c$) ; 7,40 ppm (2H, dd, $J_{b-a}$ = $J_{b-c}$ = 7,3 Hz, $H_b$) ; 7,60 ppm (2H, m, $H_d$) ; 7,76 ppm (2H, d, $J_{a-b}$ = 7,3 Hz, $H_a$)

L'amide de Weinreb ainsi obtenue (1,4 g, 3,31 mmoles) est dissoute dans 30 mL de THF anhydre, refroidie par un bain de glace et 1,25 équivalent de $LiAlH_4$ (162,3 mg, 4,14 mmoles) est alors additionné par petites fractions sur une période de 10 minutes. Le milieu réactionnel est agité pendant 40 minutes à 0°C puis hydrolysé par une solution aqueuse à 5% de $KHSO_4$ (5 mL). Le produit est extrait par l'éther diéthylique (3 X 30mL) et les phases organiques rassemblées, séchées sur $MgSO_4$ et évaporées sous vide pour conduire au Fmoc-leucinal (794 mg, 2,35 mmoles) qui est utilisé sans purification ultérieure.

**c- Synthèse sur support solide:**

Le pseudohexapeptide est synthétisé sur un synthétiseur semi-automatique (CNRS, IBMC, Strasbourg, France) à partir d'une résine Fmoc-Ser(tBu)-Wang PS réticulée par 1% de divinylbenzène (Senn Chemicals, Dielsdorf Suisse). La stratégie utilisée est un protocole classique Fmoc/tBu. L'élongation de la chaîne peptidique est réalisée à partir de 0,5 grammes de résine substituée à 0,5 méq./g par couplage successifs des Fmoc-aminoacides (0,75 mmole) , les chaînes latérales de l'asparagine et de la thréonine étant respectivement protégées par un groupe trityle et ter-butyle. Les catalyseurs de couplage sont le tétrafluoroborate de 2-(1H-benzotriazole-1-yl)-1,1,3,3-tetraméthyluronium (TBTU), (0,75 mmole), le 1-hydroxybenzotriazole (HOBt) (0,75 mmole) et la diisopropyléthylamine (DIEA) (2,25 mmoles) dans la diméthylformamide (DMF, 5mL).

L'avancement de chaque étape est contrôlé par un test colorimétrique à l'acide 2,4,6-trinitrobenzènesulfonique pour Ser, Gly, Leu, Asn, Thr et au chloranil (tétrachloro-1,4-benzoquinone) pour Pro. La déprotection N-terminale du groupe Fmoc est réalisée par une solution de pipéridine à 20% dans la DMF.

**d-Synthèse de la liaison réduite Ψ[$CH_2$-NH] :**

Cette synthèse est résumée par le schéma 2 ci-dessous :

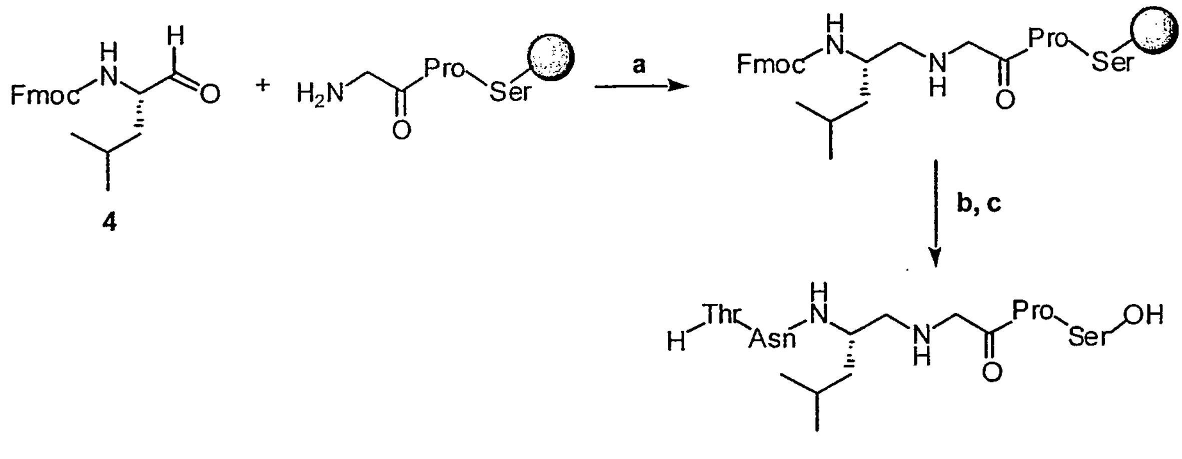


**a**: AcOH, $NaBH_3CN$ **b**: Fmoc-Xaa-OH, TBTU, BtOH, DIEA **c**: TFA, EDT, Phénol, thoianisole, $H_2O$

Schéma 2 : Synthèse de la liaison réduite Ψ[$CH_2$-NH]

Après avoir successivement couplé Fmoc-Pro-OH et Fmoc-Gly-OH et libéré la fonction -$NH_2$, l'aldéhyde Fmoc-Leu-H (0,253g 0,75 mmole) est additionné dans le réacteur, solubilisé dans 5 mL de DMF. Quelques gouttes d'AcOH glacial sont ajoutées au milieu réactionnel et 3éq. de $NaBH_3CN$ sont additionnés par petites portions sur 1 h. Le mélange est laissé sous agitation pendant une nuit. Le groupement Fmoc est déprotégé dans les conditions précédemment citées.

La synthèse de l'hexapseudopeptide est terminée par les couplages successifs de Fmoc-Asn(Trt)-OH et Fmoc-Thr(tBu)-OH dans les conditions citées plus haut.

Le peptide est clivé de la résine pendant 2 heures par un mélange de 10 mL de TFA, 0,750 g de phénol, 0,25

mL d'EDT, 0,5 mL de thioanisole et 0,5 mL d'eau désionisée. Ce mélange est initialement refroidi à 0°C mais le clivage se déroule à température ambiante. Le peptide précipite par ajout d'$Et_2O$ glacé et la résine est filtrée. Le peptide resté sur le fritté est dissous au-dessus d'un ballon plein d'$Et_2O$ glacé à l'aide de TFA.. Il est ensuite concentré et lyophilisé.

Le pseudopeptide est purifié par chromatographie liquide haute performance (HPLC) réalisée sur une chaîne Hitachi-Merck équipée d'une pompe L6200 couplée à un détecteur Jasco 875 UV. La colonne préparative utilisée est une Waters DELTA PAK C18 (300 X 7,8 mm i.d., taille des particules: 15 μm, porosité : 300 Å). L'éluant est composé d'une solution A de 0,1 % en volume de TFA (sequencing grade, Sigma) dans l'eau Ultrapure et d'une solution B de 0,08 % de TFA et de 20 % d'eau dans l'acétonitrile (Carlo Erba). Le peptide est élué par un gradient de 20% de B dans A jusque 50% sur 30 minutes à 4mL/minute. Le peptide est collecté manuellement. Après évaporation des solvants, le peptide purifié est lyophilisé avant d'être caractérisé par spectrométrie de masse et RMN.

*m/z* [ES] théorique 573,31, expérimental 574,41 pour $[M+H]^+$

Le spectre RMN est conforme à la structure attendue.

### 2.2 Hydrazinopeptides

#### a- Protocole opératoire pour la préparation du *NβBoc-NβBoc-Nα-Z- Hydrazinoglycine*

Boc2N-N(Z)-CH2-COOH a été synthétisé suivant la méthode décrite par N. Brosse et al ( N. Brosse, M.-F. Pinto, J. Bodiguel, B. Jamart-Grégoire J. Org. Chem., 2001, 66, 2869-2873), cette voie de synthèse étant résumée sur le schéma 3 ci-dessous :

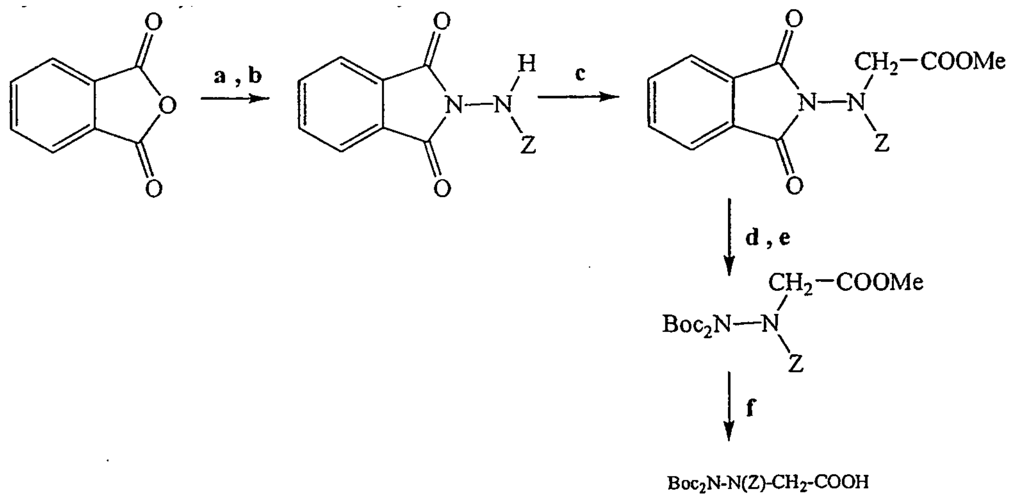


a) $H_2N$-NH-Z, THF, rt; b) DCCI, BtOH, rt; c) HO-$CH_2$-COOMe, DIAD, $PPh_3$, THF, rt;
d) $MeNH_2$, THF, rt; e) $Boc_2O$, DMAP, THF, rt; f) LiOH, THF

Schéma 3 : Synthèse du NβBoc-NβBoc-Nα-Z-Hydrazinoglycine

#### b- Synthèse sur support solide:

Cette synthèse est résumée sur le schéma 4 ci-dessous.

Le pseudohexapeptide est synthétisé sur un synthétiseur semi-automatique (CNRS, IBMC, Strasbourg, France) à partir d'une résine Fmoc-Ser(tBu)-Wang PS réticulée par 1% de divinylbenzène (Senn Chemicals, Dielsdorf Suisse). La stratégie utilisée est un protocole classique Boc/Bzl. L'élongation de la chaîne peptidique est réalisée à partir de 0,5 grammes de résine substituée à 0,69 méq./g par couplage successifs des Boc-aminoacides (1.04mmole) , les chaînes latérales de l'asparagine et de la thréonine étant respectivement protégées par un groupe xanthyle et Bzl. La *N*β,*N*β-Boc-*N*α(Z)Gly-OH est incorporée comme un acide aminé normal. Pour ce résidu, le temps de couplage est porté à une nuit au lieu des deux heures de réaction pour les couplages des autres aminoacides. Les catalyseurs de couplage sont le tétrafluoroborate de 2-(1H-benzotriazole-1-yl)-1,1,3,3-tetraméthyluronium (TBTU), (1.04 mmole), le 1-hydroxybenzotriazole (HOBt) (1.04 mmole) et la diisopropyléthylamine (DIEA) (3.12 mmoles) dans la N,N-diméthylformamide (DMF, 5mL).

L'avancement de chaque étape est contrôlé par un test colorimétrique à l'acide 2,4,6-trinitrobenzènesulfonique

pour Ser, Gly, Leu, Asn, Thr et au chloranil (tétrachloro-1,4-benzoquinone) pour Pro. La déprotection N-terminale du groupe Fmoc est réalisée par une solution de pipéridine à 20% dans la DMF.

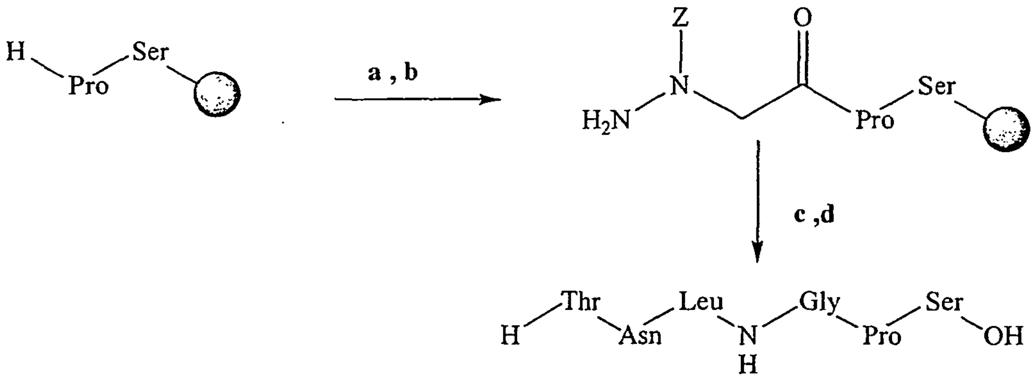


a) $Boc_2N\text{-}N(Z)\text{-}CH_2\text{-}COOH$, TBTU, BtOH, DIEA, DMF, b) TFA, c) Boc-Xaa-OH, TBTU, BtOH, DIEA, DMF; d) TFMSA, TFA, EDT, Thioanisole.

Schéma 4 : synthèse de l'hydrazino peptide

Après le couplage de la thréonine terminale, le peptide est clivé de la résine par un mélange de TFA (10 mL) et de TFMSA (1 mL) en présence de thioanisole (1mL) et d'EDT (0,5mL). Le pseudopeptide est purifié par chromatographie liquide haute performance (HPLC) réalisée sur une chaîne Hitachi-Merck équipée d'une pompe L6200 couplée à un détecteur Jasco 875 UV. La colonne préparative utilisée est une Waters DELTA PAK C18 (300 X 7,8 mm i.d., taille des particules: 15 μm, porosité : 300 Å). L'éluant est composé d'une solution A de 0,1 % en volume de TFA (sequencing grade, Sigma) dans l'eau Ultrapure et d'une solution B de 0,08 % de TFA et de 20 % d'eau dans l'acétonitrile (Carlo Erba). Le peptide est élué par un gradient de 20% de B dans A jusque 50% sur 30 minutes à 4mL/minute. Le peptide est collecté manuellement. Après évaporation des solvants, le peptide purifié est lyophilisé avant d'être caractérisé par spectrométrie de masse et RMN

**2.3. Cétométhylèneamino-peptides $\Psi$[CO-$CH_2$-NH] :**

**a- Synthèse du diméthyldioxirane (DMD) :**

Dans un ballon de 1L sont additionnés 254 mL d'eau distillée, 192 mL d'acétone et 58 g de $NaHCO_3$. Le mélange est porté à 5°C et l'addition de 120 g d'Oxone® se fait par petites portions toutes les 3 min. Un important dégagement gazeux est observé à chaque addition de l'oxydant. Lorsque l'addition est terminée, le bain froid est retiré et le DMD est récupéré par transfert sur paroi froide sous vide léger. La solution (≈ 150 mL à 0,09M) est conservée sur tamis moléculaire 4A à -20°C et utilisée dans les 24 h.

**b- Oxydation par utilisation de DMD :**

Synthèse du glyoxal Fmoc-Leu-CHO :

Le diazo Fmoc-Leu-CH=$N_2$ (548 mg, 1,5 mmol) est mis à réagir directement par solubilisation dans la solution de DMD (50 mL, 4,5 mmol). Après 10 min. d'agitation à 0°C, le solvant est évaporé et le résidu est repris dans le DCM (15 mL) afin de retirer par décantation l'eau résiduelle. Le solvant est réévaporé et le rendement est quantitatif. Le glyoxal est utilisé sans purification ultérieure et sans attendre.

Une fois la synthèse terminée, le pseudopeptide cétométhylèneamino est clivé de la résine suivant le protocole habituel.

Cette voie de synthèse est résumée sur le schéma 5 ci-dessous et est inspiré de Groarke M., Hartzoulakis B., McKervey M. A., Walker B., Williams C. H., Bioorg. Med. Chem. Lett., 2000, 10, 153-155:

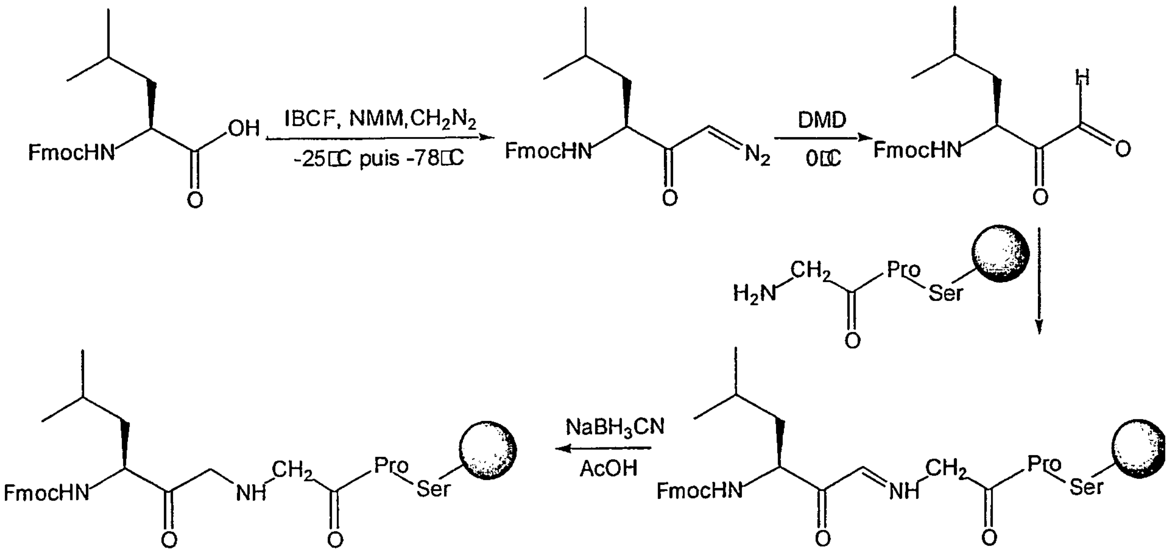


Schéma 5 : Synthèse de peptides à fonction cétométhylèneamino Ψ[CO-$CH_2$-NH]

## 2.4 Carbonylhydrazone-peptides Ψ[CO-NH-N=] :

Cette voie de synthèse est résumée sur le schéma 6 ci-dessous et est inspiréé de Lourak M., Vanderesse R., Vicherat A., Jamal-Eddine J., Marraud M., Tetrahedron Lett., 2000, 8773-8776:

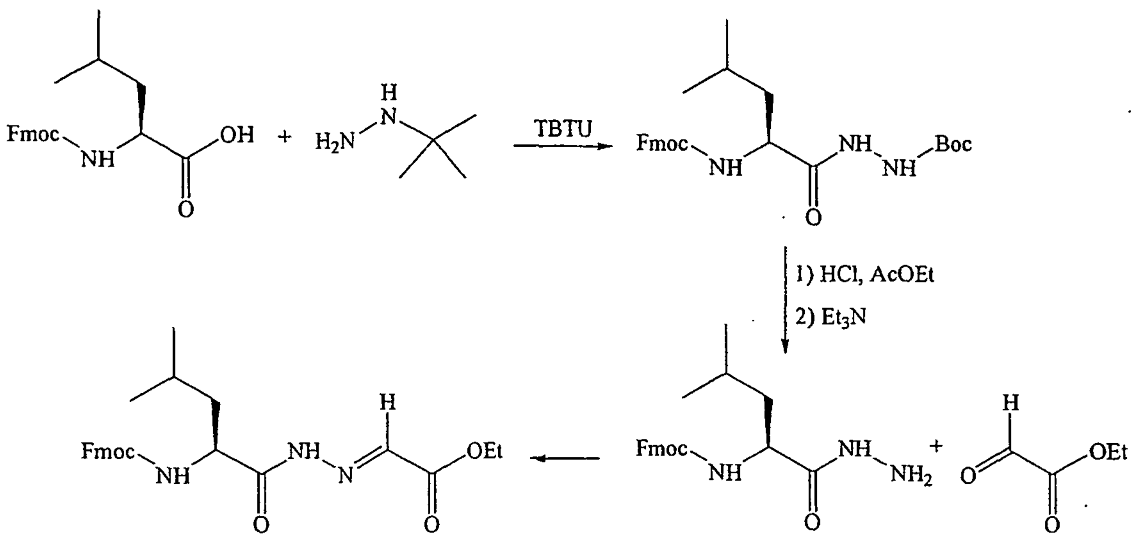


Schéma 6 : Synthèse de peptides à fonction carbonylhydrazone Ψ[CO-NH-N=]

La *N*-Fmoc leucine (1 g, 2,83 mmol) est couplée avec le *tert*-butylcarbazate (273 mg, 3,11 mmol) *via* formation d'un ester activé au TBTU dans le DCM en présence de DIEA. Le composé diprotégé est obtenu avec un rendement de 98%. La protection Boc, labile en milieu acide, est retirée par agitation du composé dans une solution de HCl 3N

dans l'acétate d'éthyle durant une heure. L'hydrazine est ensuite régénérée par action d'une solution de triéthylamine ($Et_3N$) dans le méthanol sur le chlorhydrate. Cette réaction est quantitative et propre. Le lien carbonylhydrazone est obtenu par condensation de l'hydrazine sur un mime commercial de la glycine, le glyoxylate d'éthyle (1,7 g, 16,64 mmol), pour partenaire cétonique. Aucune base n'est nécessaire à l'aboutissement de cette réaction. Un temps de réaction de 2 heures est suffisant dans le DCM. Le pseudodipeptide diéthylester est purifié sur gel de silice avec un éluant composé de 30% d'éther de pétrole dans l'acétate d'éthyle, et récupéré sous forme solide avec un rendement de 84%.

L'ester Fmoc-LeuΨ[CO-NH-N=]-Gly-OEt (1,05 g, 2,33 mmol) est solubilisé dans un mélange MeOH/THF 1/2 (v/v) à 0°C. 2 équivalents de LiOH (112 mg, 4,66 mmol) sont alors lentement additionnés et le soluté est laissé à agiter durant 10 min. Après évaporation du mélange de solvants, le résidu est repris dans l'AcOEt et traité par lavages avec une solution aqueuse de $KHSO_4$, 5% (2 X 10 mL), et de l'eau distillée (2 X 10 mL). Après séchage sur $MgSO_4$ et évaporation du solvant, l'acide obtenu (635 mg, 1,5 mmol) est engagé sans attendre dans le couplage sur une nuit avec l'hexapeptide en formation en présence de BtOH, TBTU et DIEA, comme illustré par le schéma 7.

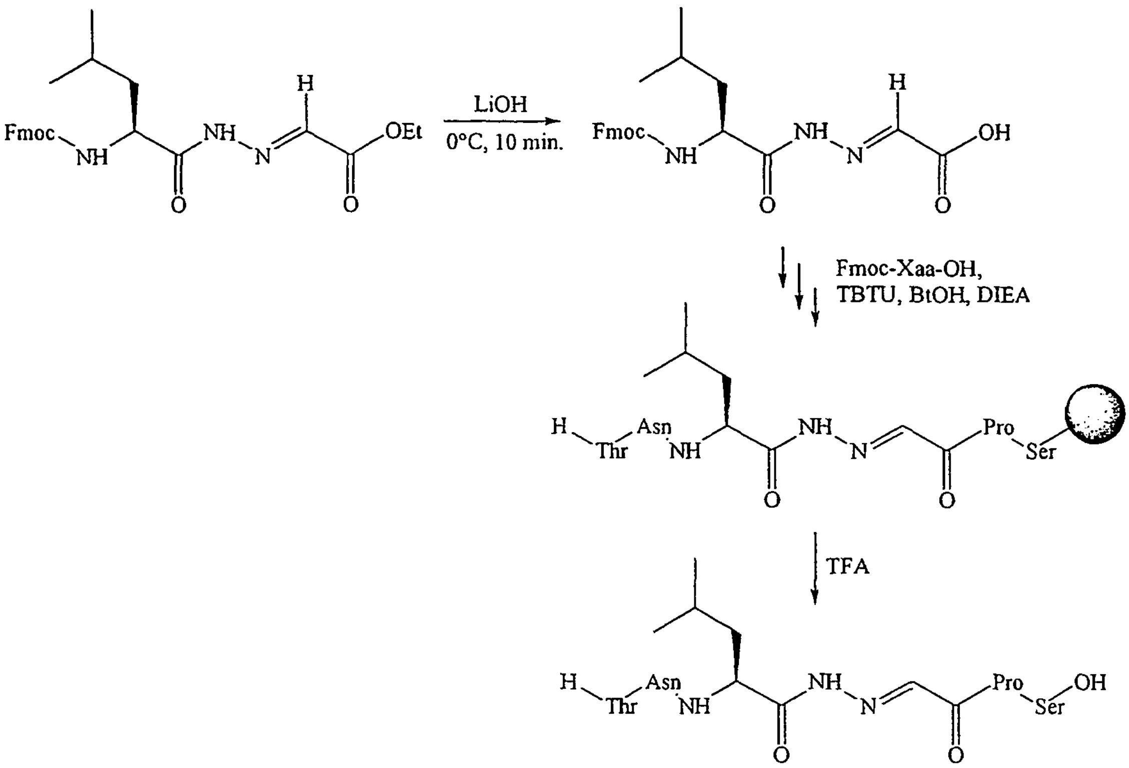


Schéma 7 : synthèse du peptide à fonction carbonyl hydrazone

Une fois la synthèse terminée, le pseudopeptide carbonylhydrazone est clivé de la résine suivant le protocole habituel.

## 3. Peptides biotinylés et/ ou porteur d'un groupement parabenzoylphénylalanine

### Synthèse de Biot-Ava-TVT-Bpa-KF :

La résine Fmoc-Phe-Wang (500 mg) est solvatée dans 5 mL de DMF. Après l'étape de déprotection utilisant 3 fois 5 mL de pipéridine à 20% dans la DMF, la Fmoc-Lys(Boc)-OH (513 mg, 3 éq.) dissoute dans 5 mL de DMF est additionnée en présence de TBTU (351 mg, 3 éq.), BtOH (168 mg, 3 éq.) et DIEA (0,6 mL, 9 éq.). Après 40 minutes d'agitation, un test est réalisé sur un prélèvement de billes de résine dans le méthanol en présence de TNBSA. Le test étant négatif (observation d'une coloration blanche des billes), l'étape de déprotection est amorcée. Ensuite, le Bpa (492,4 mg, 3 éq.) est additionné à son tour et ainsi de suite jusqu'à l'acide amino-valérique Fmoc-Ava-OH. Après déprotection du groupement Fmoc, la biotine (Bachem, Suisse) (268 mg, 3 éq.) est additionnée enfin, juste en présence de DIEA (0,6 mL, 3 éq.). L'agitation se poursuit pendant une nuit. Après rinçage de la résine par 5 X 5 mL de DCM, La

résine est séchée sous vide. Le peptide et sa résine sont mis à réagir avec un mélange contenant 0,75 g de phénol, 0,5 mL de thioanisole, 0,5 mL d'eau osmosée, 0,25 mL d'EDT et 10 mL de TFA. Si l'addition du mélange se fait dans un bain de glace à 0°C, l'agitation se poursuit 1h30 à température ambiante. Le peptide précipite par ajout d'$Et_2O$ glacé et la résine est filtrée. Le peptide resté sur le fritté est dissous au-dessus d'un ballon plein d'$Et_2O$ glacé à l'aide de TFA. Il est ensuite concentré et lyophilisé.

Les peptides sont purifiés par chromatographie liquide haute performance (HPLC). La colonne préparative utilisée est une Waters DELTA PAK C18 (15 µm, 300 Å, 7,8 x 300 mm). L'éluant est composé d'une solution A de 0,1 % en volume de TFA dans l'eau et d'une solution B de 0,08 % de TFA et de 20 % d'eau dans l'acétonitrile.

## B-ACTIVITE BIOLOGIQUE

Figures :

La figure la représente l'évolution du rapport V0/Vi caractéristique d'une inhibition impliquant un seul site de l'enzyme, La figure 1b représente l'évolution du rapport V0/Vi caractéristique d'une inhibition parabolique en accord avec le schéma réactionnel représenté sur la figure 1c.

### 1. Enzymes

Le protéasome 26S de Xénope (*Xenopus laevis*) a été purifié selon le protocole décrit dans : GLICKMAN et COUX (2001) Current Protocols in Protein Science, Suppl. 24, Wiley, New York, pp. 21.5.1-21.5.17.

Les protéasomes 26S et 20S de levure (*Saccharoyces cerevisae*) ont été purifiés selon le protocole décrit dans : LEGGETT et al (2002) Molecular Cell, 10, pp 495-507.

### 2. Substrats

Les activités peptidasiques ont été déterminées à l'aide des substrats fluorogènes Suc-LLVY-amc (CT-L), Z-LLE-βNA (PA) et Boc-LRR-amc (T-L), fournis par la société Bachem (Voisins-le-Bretonneux, France).

### 3. Appareillage

Les activités enzymatiques ont été mesurées en utilisant le fluorimètre lecteur de plaques multi-puits BMG Fluostar, piloté par Biolise. Cet appareillage est équipé d'un dispositif de thermostatisation par effet Pelletier.

Le pH des tampons a été mesuré à l'aide d'un pH-mètre pH-stat Radiometer TT1C équipée d'une électrode de type B.

Les traitements mathématiques et statistiques des données cinétiques ont été réalisés grâce au logiciel Kaleidagraph 3.08.d (Abelbeck Software).

### 4. Mesure des activités du protéasome

Les activités peptidasiques des protéasomes 26S de levure et de xénope et celles du protéasome 20S de levure latent et activé ont été déterminées dans les conditions décrites dans le tableau II.

**Tableau II.** Conditions de mesures des activités peptidasiques des différentes catégories d'enzymes.

| Protéasome | Activité | Substrat (concentration) | Concentration de l'enzyme (µg/mL) | Tampon |
|---|---|---|---|---|
| 26S | CT-L | Suc-LLVY-amc (100 µM) | 1,5 | TrisHCl 20 mM pH 7,5, |
| | T-L | Boc-LRR-amc (200 µM) | 3 | DTT 1 mM, $MgCl_2$ 1 mM |
| | PA | Z-LLE-βNA (200 µM) | 3 | ATP 1 mM, glycérol 10 % |

(suite)

| Protéasome | Activité | Substrat (concentration) | Concentration de l'enzyme (μg/mL) | Tampon |
|---|---|---|---|---|
| **20S latent** | CT-L | Suc-LLVY-amc (100 μM) | 30 | TrisHCl 20 mM pH 7,5, |
| | T-L | Boc-LRR-amc (200 μM) | 60 | DTT 1 mM, glycérol 10 % DTT 1 mM, glycérol 10 % |
| | PA | Z-LLE-βNA (200 μM) | 60 | |
| **20S activé** | CT-L | Suc-LLVY-amc (100 μM) | 15 | Tris HCl 20 mM pH 7,5, |
| | PA | Z-LLE-βNA (200 μM) | 30 | DTT 1 mM, glycérol 10 %, SDS 0,02 % |
| CT-L : activité de type chymotrypsique ; T-L : activité de type trypsique ; PA : activité de type post-acide (ou caspasique) | | | | |

## 5. Détection et étude des effets inhibiteurs

Les composés étudiés sont solubilisés dans le tampon (peptides, pseudopeptides), ou dans le DMSO (lipopeptides, peptides photoactivables). L'enzyme est pré-incubée (15 min à 30 °C) dans le tampon correspondant (tableau II), en présence de l'inhibiteur. Pour les cas où l'inhibiteur est solubilisé dans le DMSO (lipopeptides, peptides photoactivables), le contrôle sans inhibiteur contient une quantité de DMSO identique à celle des essais avec inhibiteur (3,5 %, v/v). La réaction est déclenchée par l'ajout du substrat. Elle est suivie de façon continue pendant 30 min à 30 °C. Les vitesses initiales des essais avec inhibiteurs (calculées à partir des points expérimentaux) sont comparées à celles des contrôles. Les résultats présentés ont été obtenus en calculant la moyenne d'au moins deux essais indépendants. La variabilité est inférieure à 10 %.

### 5.1- Analyses cinétiques

Le paramètre $IC_{50}$ correspond à la concentration d'inhibiteur conduisant à une perte d'activité enzymatique de 50 %.

#### a. Détermination du paramètre $IC_{50}$

L'enzyme est pré-incubée en présence de concentrations croissantes d'inhibiteur. La réaction est déclenchée par l'ajout du substrat (voir le paragraphe « Détection et étude des effets inhibiteurs »). Le pourcentage d'inhibition est calculé à partir de l'équation 1.

$$\%\,\text{Inhibition} = 100.\frac{(V_0 - V_i)}{V_0} \qquad \text{éq. 1}$$

dans laquelle $V_0$ est la vitesse du contrôle, $V_i$ la vitesse en présence d'inhibiteur.

Les points expérimentaux décrivent l'évolution de l'effet inhibiteur du composé étudié en fonction de sa concentration. En règle générale, ils s'ajustent à la courbe décrite par l'équation 2 dans laquelle [I] est la concentration d'inhibiteur.

$$\%\,\text{Inhibition} = \frac{100.[I]}{\text{IC}_{50} + [I]} \qquad \text{éq. 2}$$

Lorsque l'inhibition est coopérative, les points expérimentaux s'ajustent à la courbe décrite par l'équation 3 dans laquelle n représente l'indice de coopérativité.

$$\%Inhibition = \frac{100.[I]^n}{IC_{50}{}^n + [I]^n}$$

b. Etude du mécanisme d'inhibition

Le mécanisme de l'inhibition est déterminé en traçant la cou éq. 3 l'évolution du rapport $V_0/V_i$ en fonction de la concentration d'inhibiteur.

• *Inhibition compétitive stricte*

Dans le cas d'une inhibition impliquant un seul site de l'enzyme, l'évolution du rapport $V_0/V_i$ en fonction de la concentration d'inhibiteur est une droite (figure la) définie par l'équation 4.

$$\frac{V_0}{V_i} = 1 + \frac{[I]}{K_{iapp}} \qquad \text{éq. 4}$$

C'est le cas lorsque l'inhibition est compétitive stricte : PAPAPOSTOLOU et al, Biochem. Biophys. Res. Comm., 2, 295, 1090-1095 (2002); STEIN et al, Biochemistry, 35, 3899-3908(1989, avec :

$$K_{iapp} = K_i + \frac{[S]}{K_m} \qquad \text{éq. 5}$$

• *Inhibition parabolique*

Lorsque l'inhibition implique deux sites distincts de l'enzyme, l'évolution du rapport $V_0/V_i$ en fonction de la concentration d'inhibiteur décrit une parabole (figure 1b) définie par l'équation 6, en accord avec le schéma réactionnel de la figure 1c.

$$\frac{V_0}{V_i} = 1 + \frac{[I]}{K_{i1app}} + \frac{[I]^2}{K_{i1app}.K_{i2app}} \qquad \text{éq. 6}$$

Dans le cas de l'inhibition des activités CT-L et PA, le premier site est un site catalytique, alors que le deuxième serait un site régulateur non-catalytique, dont la localisation est inconnue : PAPAPOSTOLOU et al. Biochem. Biophys. Res. Comm., 2, 295, 1090-1095 (2002) ; KISSELEV et al., J. Biol. Chem., 278, 35869-35877, (2003).

**6- Exemples**

**6.1. Peptides**

A titre comparatif, on a étudié différents peptides qui sont des inhibiteurs de l'activité CT-L et de l'activité post-acide du protéasome 20S activé. On peut citer à titre d'exemple les peptides TVTFKF (activité CT-L : $IC_{50}$ = 229 μM ; activité PA : $IC_{50}$ = 210 μM) et TITYKF (activité CT-L : $IC_{50}$ = 260 μM ; activité PA : $IC_{50}$ = 336 μM). Ils agissent à la fois sur les sites actifs du protéasome et les sites régulateurs (cinétiques paraboliques).

**6.2. Lipopeptides**

Plusieurs lipopeptides sont des inhibiteurs de l'activité CT-L du protéasome 20S activé.
L'effet inhibiteur est fonction de la séquence du peptide et de la longueur de la chaîne aliphatique. On désigne par CX une chaîne $CH_3$-$(CH_2)_x$-CO-

**Tableau III :** Effet inhibiteur des lipopeptides sur l'activité CT-L du protéasome 20S activé de levure, après traitement par 35 μM de lipopeptide (17,5 μM pour C18/TVTYKF).

| | C6 | C8 | C10 | C12 | C14 | C16 | C18 |
|---|---|---|---|---|---|---|---|
| TITFDY | 37 % | 32% | 35 % | 14 % | 6% | 20% | 34 % |
| TVTYKF | 20 % | 50 % | 22 % | 10 % | 0 % | | |
| TVTFKF | 32 % | 10 % | 42 % | | | | |

. Des $IC_{50}$ de l'ordre de 35 μM sont observés pour les lipopeptides $CH_3-(CH_2)_6$-CO-TVTYKF et $CH_3-(CH_2)_8$-CO-TVTFKF. La chaîne carbonée en C10 lorsqu'elle est fixée sur l'extrémité N-terminale du peptide TVTFKF augmente la capacité inhibitrice d'un facteur 6,5 (comparaison entre $CH_3-(CH_2)_8$-CO-TVTFKF et le peptide TVTFKF). De même, une augmentation d'un facteur 17 est observée par modification de l'extrémité N-terminale de TVTYKF par la chaîne carbonée en C8.

. Pour un peptide de séquence donnée, l'effet inhibiteur est en général très sensible à la longueur de la chaîne carbonée, suggérant que des modulations précises de l'effet inhibiteur puissent être obtenues en jouant simplement sur ce paramètre. La chaîne aliphatique lipophile est donc bien susceptible de renforcer l'effet inhibiteur du peptide correspondant.

## 6.2. Pseudopeptides

Le peptide ci-dessous a été synthétisé :

TNLGPS

La séquence TNLGPS a ensuite été utilisée comme point de départ de la synthèse d'une série de pseudopeptides.

Le lien pseudopeptidique amide réduit -Ψ[$CH_2$-NH]- est introduit entre les résidus leucine et glycine. Cette liaison est non hydrolysable.

TNL-Ψ[$CH_2$-NH]-GPS (**1**)

Ac-TNL-Ψ[$CH_2$-NH]-GPS (**2**)

Le pseudopeptide correspondant TNL-Ψ[$CH_2$-NH]-GPS (1) se comporte comme un inhibiteur du protéasome 20 S activé. Les valeurs estimées de l'$IC_{50}$ de ce pseudopeptide est de 380 μ M alors que le peptide TLNGPS inhibe le protéasome avec une $IC_{50}$ de 1750 μM (test dans des conditions expérimentales où son hydrolyse est négligeable). L'analyse cinétique montre que le pseudopeptide **1** réagit avec les sites catalytiques et le(s) site(s) régulateur(s).

Le pseudopeptide **2** obtenu par acétylation de l'extrémité N-terminale de 1 est deux fois moins efficace que **1**.

On retrouve le même ordre d'efficacité inhibitrice vis-à-vis de l'activité post-acide PA : 63 % pour [1] = 500 μM ; 28 % pour [2] = 1 mM.

## 6.3. Peptides biotinylés et/ ou porteur d'un groupement *para*-benzoyl-phénylalanine

Cette catégorie est exemplifiée par la molécule :

Biot-Ava-TVT-Bpa-KF (3)

$IC_{50}$ = 32 μM

Elle présente un groupe réactionnel photoactivable parabenzoylphénylalanine et un groupement Bpa (Biot = biotinyl et Ava = acide δ-aminovalérique).

**7- Effet activateur du protéasome :**

**7.1- Détection et quantification des effets activateurs :**

Les composés étudiés sont solubilisés dans le tampon ou dans le DMSO. L'enzyme est pré-incubée (15 minutes à 30°C) dans le tampon correspondant (tableau II), en présence de la molécule à tester. Dans le cas où la molécule est solubilisée dans le DMSO, le contrôle (pas d'addition de molécule à tester) contient une quantité de DMSO identique à celle des essais (3,5% v/v). La réaction est déclenchée par l'ajout du substrat. Elle est suivie de façon continue pendant 30 minutes à 30°C. Les résultats présentés ont été obtenus en calculant la moyenne d'au moins deux essais indépendants. Une activation se caractérise par une activité après traitement par la molécule testée supérieure à 100%. La variabilité est inférieure à 10%. Les résultats sont exprimés à l'aide d'un facteur d'activation $f_a$ égal au rapport entre la vitesse initiale $V_a$ en présence du composé testé et la vitesse initiale du témoin $V_0$.

**7.2. Résultats :**

Plusieurs peptides et lipopeptides sont des activateurs de l'activité CT-L et/ou de l'activité T-L du protéasome 20S latent.

| Peptide/Lipopeptide | $f_a$ Activité CT-L | $f_a$ Activité T-L |
|---|---|---|
| TITFDY | 5 | 3 |
| TVTFKF | 2,3 | 1,7 |
| TITYEY | 2 | - |
| TITYDF | - | 2,5 |
| $CH_3-(CH_2)_{16}$-CO-TITFDY | 6 | 1,2 |
| $CH_3-(CH_2)_{14}$-CO- TITFDY | 3 | - |
| $CH_3-(CH_2)_{16}$-CO-TVTYKF | 3,2 | - |
| $CH_3-(CH_2)_{14}$-CO- TVTYKF | 2 | - |
| $CH_3-(CH_2)_{12}$-CO- TVTYKF | 2 | - |

Peptides et lipopeptides constituent donc des molécules pouvant moduler en finesse l'activité CT-L grâce à des changements dans la longueur de la chaîne aliphatique. La complexité des effets doit être liée à la multiplicité des sites possibles d'interaction, sites actifs ou sites régulateurs.

## Revendications

**1.** Molécule qui répond à la formule

TNL*GPS,

dans laquelle * représente :

- une liaison choisie parmi les liaisons ester, thioester, cétométhylène, cétométhylèneamino, N-méthylamide, amide inversée, Z/E vinylène, éthylène, méthylènethio, méthylèneoxy, thioamide, méthylène amino, hydrazino, carbonylhydrazone et N-amino
ou
- la présence d'un aza-acide aminé en substitution de l'un des acides aminés adjacents à *, les acides aminés étant de configuration L ou D.

**2.** Molécule, **caractérisée en ce qu'**elle comporte une molécule selon la revendication 1 couplée sur son extrémité C-terminale et/ou sur son extrémité N-terminale, avec une autre molécule qui favorise sa biodisponibilité.

**3.** Médicament, **caractérisé en ce qu'**il comporte une molécule selon l'une quelconque des revendications 1 et 2, dans un support pharmaceutiquement acceptable.

**4.** Utilisation d'une molécule selon l'une quelconque des revendications 1 et 2, pour la préparation d'un médicament destiné à la prévention et au traitement d'une pathologie impliquant le protéasome.

**5.** Utilisation selon la revendication 4, **caractérisée en ce que** la pathologie est sélectionnée parmi : les cancers impliquant des tumeurs hématologiques ou des tumeurs solides, les maladies auto-immunes, le SIDA, les maladies inflammatoires, les pathologies cardiaques et les conséquences des processus ischémiques que ce soit au niveau myocardique, cérébral ou pulmonaire, le rejet de d'allogreffe, la fonte musculaire, les accidents vasculaires cérébraux, les traumatismes, les brûlures.

**6.** Utilisation selon la revendication 4, pour la préparation d'un médicament destiné à la radiosensibilisation d'une tumeur.

**7.** Composition cosmétique et/ou dermatologique comprenant une molécule selon l'une quelconque des revendications 1 et 2 dans un support cosmétiquement et/ou dermatologiquement acceptable.

## Claims

**1.** A molecule corresponding to the formula

TNL*GPS,

in which * represents:

- a bond selected from the ester, thio ester, ketomethylene, ketomethylene amino, N-methylamide, inverse amide, Z/E vinylene, ethylene, methylenethio, methyleneoxy, thioamide, methyleneamino, hydrazino, carbonyl hydrazone and N-amino bonds
or
- the presence of an aza-amino acid as a substitute for one of the amino acids adjacent to *, the amino acids being of L or D configuration.

**2.** A molecule, **characterised in that** it comprises a molecule according to claim 1 coupled, at its C-terminal end and/or at its N-terminal end, with another molecule which promotes its bioavailability.

3. A medicament, **characterised in that** it comprises a molecule according to any one of claims 1 and 2 in a pharmaceutically acceptable carrier.

4. The use of a molecule according to any one of claims 1 and 2 for preparing a medicament for use in the prevention and treatment of a pathology involving the proteasome.

5. The use according to claim 4, **characterised in that** the pathology is selected from: cancers involving haematological tumors or solid tumors, autoimmune diseases, AIDS, inflammatory diseases, cardiac pathologies and the consequences of ischaemic processes whether at the myocardial, cerebral or pulmonary level, allograft rejection, muscular atrophy, strokes, traumas and burns.

6. The use according to claim 4 for preparing a medicament for use in the radiosensitisation of a tumor.

7. A cosmetic and/or dermatological composition comprising a molecule according to any one of claims 1 and 2 in a cosmetically and/or dermatologically acceptable carrier.

**Patentansprüche**

1. Molekül, das der Formel

TNL*GPS

entspricht, in der *:

- eine Bindung, ausgewählt unter Ester-, Thioester-, Ketomethylen-, Ketomethylenamino-, N-Methylamid-, inversen Amid-, Z/E-Vinylen-, Ethylen-, Methylenthio-, Methylenoxy-, Thioamid-, Methylenamino-, Hydrazin-, Carbonylhydrazon- und N-Amino-Bindungen, oder
- das Vorliegen einer Azaaminosäure als Substitution einer der Aminosäuren, die zu * benachbart sind, wobei die Aminosäuren die Konfiguration L oder D haben, darstellt.

2. Molekül, **dadurch gekennzeichnet, dass** es ein Molekül nach Anspruch 1 umfasst, das an seinem C-terminalen Ende und/oder an seinem N-terminalen Ende mit einem anderen Molekül gekoppelt ist, welches seine Bioverfügbarkeit begünstigt.

3. Medikament, **dadurch gekennzeichnet dass** es ein Molekül nach einem der Ansprüche 1 und 2 in einem pharmazeutisch verträglichen Träger umfasst.

4. Verwendung eines Moleküls nach einem der Ansprüche 1 und 2 für die Herstellung eines Medikaments, das zur Prävention und Behandlung einer Pathologie, bei der das Proteasom involviert ist, bestimmt ist.

5. Verwendung nach Anspruch 4, **dadurch gekennzeichnet, dass** die Pathologie ausgewählt ist aus: Krebsarten, die hämatologische Tumore oder solide Tumore implizieren, Autoimmunerkrankungen, Aids, Entzündungserkrankungen, Herzerkrankungen und Folgen ischämischer Prozesse wie auf myokardialem, zerebralem oder pulmonalem Niveau, Homotransplantatabstoßung, Muskelschwund, zerebrovaskuläre Ereignisse, Traumata, Verbrennungen.

6. Verwendung nach Anspruch 4 für die Herstellung eines Medikaments, das zur Radiosensibilisierung eines Tumors bestimmt ist.

7. Kosmetische und/oder dermatologische Zusammensetzung, die ein Molekül nach einem der Ansprüche 1 und 2 in einem kosmetisch und/oder dermatologisch verträglichen Träger umfasst.

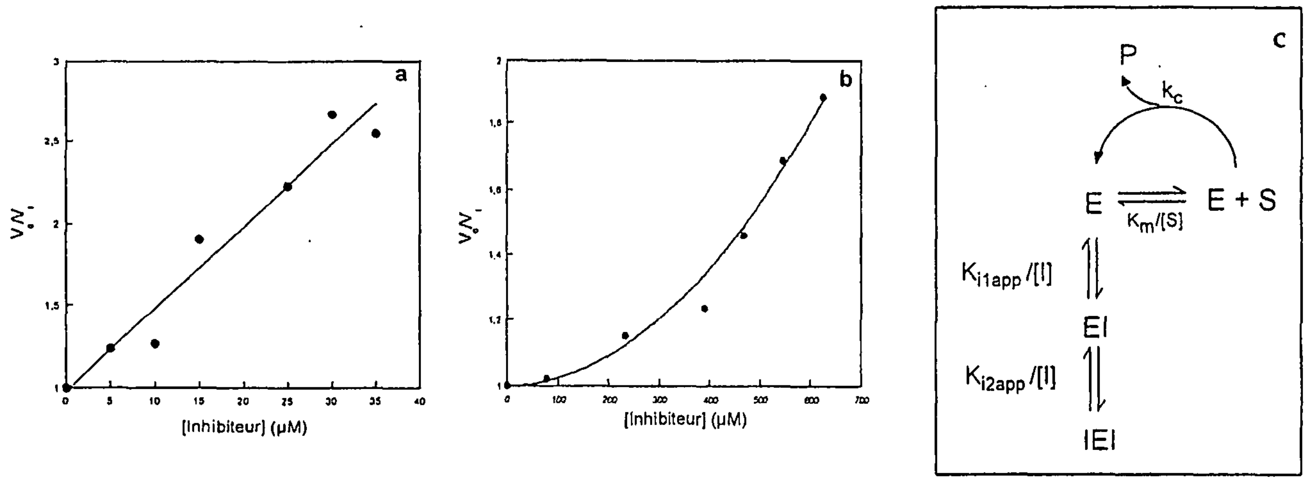


**FIGURE 1 :** Evolution du rapport V0/Vi caractéristique (a) d'une inhibition impliquant un seul site de l'enzyme, (b) d'une inhibition parabolique en accord avec le schéma réactionnel (c).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5847076 A
- JP 2002029996 B
- WO 9833812 A
- EP 45665 A, JENNINGS-WHITE


### Littérature non-brevet citée dans la description


- **Eytan et al.** *Proc. Natl. Acad. Sci. USA,* 1989, vol. 86, 7751-7755
- **Reichsteiner et al.** *J. Biol. Chem.,* 1993, vol. 268, 6065-6068
- **Glotzer et al.** *Nature,* 1991, vol. 349, 132-138
- **Ciechanover et al.** *Proc. Natl. Acad. Sci. USA,* 1991, vol. 88, 139-143
- **Murakami et al.** *Nature,* 1992, vol. 360, 597-599
- **Brown et al.** *Nature,* 1991, vol. 355, 355-360
- **M. Reboud-Ravaux.** Progress in Molecular and Subcellular Biology. Springer Verlag, 2002, vol. 29, 109-125
- **Kisselev et al.** *Chemistry & Biology,* 2001, vol. 8, 739-758
- **Kisselev et al.** *J. Biol. Chem,* 2002, vol. 277, 22260-22270
- **Wilk et al.** *Mol. Biol. Rep.,* 1997, vol. 24, 119-124
- **Ruiz De Mena et al.** *Biochem. J.,* 1993, vol. 296, 93-97
- **Arribas et al.** *J. Biol. Chem.,* 1990, vol. 265, 13969-13973
- **Richardson et al.** *Cancer Control,* 2003, vol. 10, 361-366
- *Bioorganic and Medicinal Chemistry,* 2003, vol. 11, 4881-4889
- **Papapostolou et al.** *BBRC,* 2002, vol. 295, 1090-1095
- **ROJAS et al.** *Nat. Biotechnol.,* 1998, vol. 16, 370-375
- **FUTAKI et al.** *J. Biol. Chem.,* 2001, vol. 276, 5836-5840
- **MORRIS et al.** *Nat. Biotechnol.,* 2001, vol. 19, 1173-1176
- **SPATOLA.** *Vega Data,* 1983, vol. 1 (3
- **SPATOLA.** Chemistry and Biochemistry of Amino Acids Peptides and Proteins. Marcel Dekker, 1983, 267
- **MORLEY.J.-S.** *Trends Pharm. Sci.,* 1980, 463-468
- **HUDSON et al.** *Int. J. Pept. Prot. Res.,* 1979, vol. 14, 177-185
- **SPATOLA et al.** *Life Sci.,* 1986, vol. 38, 1243-1249
- **Hann.** *J.Chem. Soc. Perkin Trans.I,* 1982, 307-314
- **ALMQUIST et al.** *J. Med. Chem.,* 1980, vol. 23, 1392-1398
- **HOLLADAY et al.** *Tetrahedron Lett.,* 1983, vol. 24, 4401-4404
- **HRUBY et al.** *Life Sci.,* 1982, vol. 31, 189-199
- **RICHARSON et al.** *Cancer Control,* 2003, vol. 10, 361-366
- **ADAMS.** *Drug Discovery Today,* vol. 8, 307-311
- **LENZ.** *Cancer Treatment Reviews,* 2003, vol. 29, 41-48
- **ELLIOT et al.** *J. Allergy Clin. Immunol.,* 1999, vol. 104, 294-300
- **ELLIOT et al.** *Journal of Molecular Medecine,* 2003, vol. 81, 235-245
- **LECKER et al.** *J. Nutr.,* 1999, vol. 129, 227S-237S
- **SCHUBERT.** *Proc. Natl. Acad. Sci. USA,* 2000, vol. 97, 1357-1362
- **Schwartz et al.** *J. Immunol,* 2000, vol. 164, 6114-6157
- **CAMPBELL et al.** *J. Mol. Cell Cardiol.,* vol. 31, 467-476
- **ZHANG et al.** *Curr. Drug Targets Inflamm. Allergy,* 2002, vol. 1, 151-156
- **DI NAPOLI et al.** *Current Opinion Invest. Drugs,* 2003, vol. 4, 303-341
- **STRAMER et al.** *Invest. Ophthalmol. Vis. Sci.,* 2001, vol. 42, 1698-1706
- **M. Reboud-Ravaux.** Progress in Molecular and Subcellular Biology. Springer Verlag, 2002, vol. 29, 42-60
- **SIDELL et al.** *J. Neur. Chem.,* 2001, vol. 79, 510-521
- **FISHER et al.** *Photochem. Photobiol.,* 1999, vol. 69, 154-157
- **GRUNE.** *Hautartz,* 2003, vol. 54, 818-821
- **LY et al.** *Science,* 2000, vol. 287, 2486-2492

- **Neimark, J. ; Briand, J. P.** *Pept. Res.,* 1993, vol. 6, 219-228
- **N. Brosse ; M.-F. Pinto ; J. Bodiguel ; B. Jamart-Grégoire.** *J. Org. Chem.,* 2001, vol. 66, 2869-2873
- **Groarke M. ; Hartzoulakis B. ; McKervey M. A. ; Walker B. ; Williams C. H.** *Bioorg. Med. Chem. Lett.,* 2000, vol. 10, 153-155
- **Lourak M. ; Vanderesse R. ; Vicherat A. ; Jamal-Eddine J. ; Marraud M.** *Tetrahedron Lett.,* 2000, 8773-8776
- **GLICKMAN ; COUX.** Current Protocols in Protein Science. Wiley, 2001, vol. 24, 21.5.1-21.5.17
- **LEGGETT et al.** *Molecular Cell,* 2002, vol. 10, 495-507
- **PAPAPOSTOLOU et al.** *Biochem. Biophys. Res. Comm.,* 2002, vol. 2 (295), 1090-1095
- **STEIN et al.** *Biochemistry,* 1989, vol. 35, 3899-3908
- **KISSELEV et al.** *J. Biol. Chem.,* 2003, vol. 278, 35869-35877

SEQUENCE LISTING

<110> CENTRE NATIONAL DE LA RECHERCHE SCIENTIFIQUE (CNRS)

<120> NOUVEAUX MODULATEURS DU PROTEASOME

<130> VCsts644-121

<160> 38

<170> PatentIn version 3.1

<210> 1
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 1

```
Xaa Thr Val Thr Tyr Asp Tyr
1               5
```

<210> 2
<211> 7
<212> PRT
<213> Artificial sequence

<220>
<223> Molécule

<220>
<221> misc_feature
<222> (1)..(1)
<223> Xaa = Ch3-(CnH2n)-CO- avec n = 4,6,8,10,12,14,16,18

<400> 2

```
Xaa Thr Ile Ser Tyr Asp Tyr
1               5
```

<210> 3
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 3

```
Xaa Thr Val Ser Tyr Lys Phe
1               5
```

<210> 4
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 4

```
Xaa Thr Ile Thr Phe Asp Tyr
1               5
```

<210> 5
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 5

```
Xaa Thr Ile Thr Tyr Lys Phe
1               5
```

<210> 6
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 6

```
Xaa Thr Ile Thr Tyr Glu Tyr
1               5
```

<210> 7
<211> 7
<212> PRT
<213> Artificial séquence

<220>
<223> Molécule

<220>
<221> misc_feature
<222> (1)..(1)
<223> Xaa = CH3-(CnH2n)-CO- avec n = 4,6,8,10,12,14,16,18

<400> 7

```
Xaa Thr Ile Thr Tyr Asp Phe
1               5
```

<210> 8
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 8

```
Xaa Thr Val Thr Tyr Lys Leu
1               5
```

<210> 9
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 9

```
Xaa Thr Val Thr Tyr Lys Tyr
1               5
```

<210> 10
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 10

```
Xaa Thr Val Thr Phe Lys Phe
1               5
```

<210> 11
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,19,16,18

<400> 11

```
Xaa Thr Ile Thr Tyr Asp Leu
1               5
```

<210> 12
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 12

```
Xaa Thr Ile Thr Phe Asp Tyr
1               5
```

<210> 13
<211> 7
<212> PRT

<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 13

```
Xaa Thr Val Thr Phe Lys Phe
1               5
```

<210> 14
<211> 7
<212> PRT
<213> Artificial sequence

<220>
<223> Molécule

<220>
<221> misc_feature
<222> (1)..(1)
<223> Xaa = CH3-(CnH2n)-CO- avec n = 4,6,8,10,12,14,16,18

<400> 14

```
Xaa Thr Val Thr Tyr Lys Phe
1               5
```

<210> 15
<211> 8
<212> PRT
<213> Artificial sequence

<220>
<223> Molécule

<220>
<221> misc_feature
<222> (1)..(1)
<223> Xaa = groupement biotinyle

<220>
<221> misc_feature
<222> (2)..(2)
<223> Xaa = groupement acide delta amino valérique

<220>
<221> misc_feature
<222> (6)..(6)

<223> Xaa = groupement para-benzoyl-phénylalanine

<400> 15

```
Xaa Xaa Thr Val Thr Xaa Lys Phe
1               5
```

<210> 16
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature

<222> (1)..(1)
<223> Xaa = groupement biotinyle

<220>
<221> misc_feature
<222> (2)..(2)
<223> Xaa = groupement acide delta amino valérique

<400> 16

```
Xaa Xaa Thr Val Thr Xaa Lys Tyr
1               5
```

<210> 17
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 17

```
Xaa Xaa Thr Val Thr Xaa Lys Leu
1               5
```

<210> 18
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 18

```
Xaa Xaa Thr Val Thr Xaa Asp Phe
1               5
```

<210> 19
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 19

```
Xaa Xaa Thr Val Thr Xaa Asp Tyr
1               5
```

<210> 20
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 20

```
Xaa Xaa Thr Val Thr Xaa Asp Leu
1               5
```

<210> 21
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 21

```
Xaa Xaa Thr Ile Thr Xaa Lys Phe
1               5
```

<210> 22
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 22

```
Xaa Xaa Thr Ile Thr Xaa Lys Tyr
1               5
```

<210> 23
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)

<223> Xaa = groupement biotinyle

<220>
<221> misc_feature
<222> (2)..(2)
<223> Xaa = groupement acide delta amino valérique

<400> 23

```
Xaa Xaa Thr Ile Thr Xaa Lys Leu
1               5
```

<210> 24
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 24

```
Xaa Xaa Thr Ile Thr Xaa Asp Phe
1               5
```

<210> 25
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>

<221> misc_feature
<222> (1)..(1)
<223> Xaa = groupement biotinyle

<220>
<221> misc_feature
<222> (2)..(2)
<223> Xaa = groupement acide delta amino valérique

<400> 25

```
Xaa Xaa Thr Ile Thr Xaa Asp Tyr
1               5
```

<210> 26
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (2)..(2)
<223> Xaa = groupement biotinyle

<220>
<221> misc_feature
<222> (1)..(1)
<223> Xaa = groupement acide delta amino valérique

<400> 26

```
Xaa Xaa Thr Ile Thr Xaa Asp Leu
1               5
```

<210> 27
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 27

```
Xaa Xaa Thr Val Thr Xaa Glu Phe
1               5
```

<210> 28
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 28

```
Xaa Xaa Thr Val Thr Xaa Glu Tyr
1               5
```

<210> 29
<211> 8
<212> PRT
<213> Artificial sequence

<220>
<223> Molécule

<220>
<221> misc_feature
<222> (6)..(6)

<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement bacide delta amino valérique

<400> 29

```
Xaa Xaa Thr Val Thr Xaa Glu Leu
1               5
```

<210> 30
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 30

```
Xaa Xaa Thr Ile Thr Xaa Glu Phe
1               5
```

<210> 31
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 31

```
Xaa Xaa Thr Ile Thr Xaa Glu Tyr
1               5
```

<210> 32
<211> 8
<212> PRT
<213> Artificial sequence

<220>
<223> Molécule

<220>
<221> misc_feature

<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 32

```
Xaa Xaa Thr Ile Thr Xaa Glu Leu
1               5
```

<210> 33
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 33

```
Xaa Xaa Thr Val Thr Xaa Asn Phe
1               5
```

<210> 34
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 34

```
Xaa Xaa Thr Val Thr Xaa Asn Tyr
1               5
```

<210> 35
<211> 8
<212> PRT

<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 35

```
Xaa Xaa Thr Val Thr Xaa Asn Leu
1               5
```

<210> 36
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 36

```
Xaa Xaa Thr Ile Thr Xaa Asn Phe
1               5
```

<210> 37
<211> 8

<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 37

```
Xaa Xaa Thr Ile Thr Xaa Asn Tyr
1               5
```

<210> 38
<211> 8
<212> PRT
<213> Artificial sequence

<220>
<223> Molécule

<220>
<221> misc_feature
<222> (6)..(6)
<223> Xaa = groupement para-benzoyl-phénylalanine

<220>
<221> misc_feature
<222> (1)..(1)
<223> Xaa = groupement biotinyle

<220>
<221> misc_feature
<222> (2)..(2)
<223> Xaa = groupement acide delta amino valérique

<400> 38

```
Xaa Xaa Thr Ile Thr Xaa Asn Leu
1               5
```